(19) Europäisches Patentamt / European Patent Office / Office européen des brevets

(11) **EP 3 791 867 A1**

(12) **EUROPEAN PATENT APPLICATION**

(43) Date of publication:
**17.03.2021 Bulletin 2021/11**

(21) Application number: **20203011.0**

(22) Date of filing: **20.08.2013**

(51) Int Cl.:
*A61K 9/50* (2006.01)    *A61K 9/14* (2006.01)
*A61K 47/32* (2006.01)    *A61K 47/34* (2017.01)
*A61K 47/38* (2006.01)    *A61K 9/16* (2006.01)
*A61K 9/20* (2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **20.08.2012 PCT/JP2012/071016**

(62) Document number(s) of the earlier application(s) in
accordance with Art. 76 EPC:
**13830393.8 / 2 886 109**

(71) Applicant: **Sumitomo Dainippon Pharma Co., Ltd.
Chuo-ku
Osaka-shi
Osaka 541-8524 (JP)**

(72) Inventors:
• KOBIKI, Mitsuaki
Ibaraki-shi, Osaka 567-0878 (JP)
• OCHIAI, Yasushi
Ibaraki-shi, Osaka 567-0878 (JP)

(74) Representative: **J A Kemp LLP
14 South Square
Gray's Inn
London WC1R 5JJ (GB)**

Remarks:
This application was filed on 21.10.2020 as a
divisional application to the application mentioned
under INID code 62.

(54) **MEDICAMENT-CONTAINING HOLLOW PARTICLE**

(57)    The problem of the present invention is to provide a medicament-containing particle having sufficient strength durable against compression, coating and the like, and imparted with desired functionality and a process for preparation thereof and the like.

The present invention provides a particle composed of a shell and a hollow, wherein the shell contains a medicament and a polymer, and a volume ratio of the hollow relative to the whole particle is 1% - 50%. The present invention also provides a process for preparation of the above hollow particle, which includes a step of granulating a powder mixture containing a medicament and a polymer, while spraying a solvent capable of dissolving the polymer.

Fig. 1-1

Example 1-1  spherical particles
containing 90% of Compound A (X250)

Fig. 1-2

Example 1-2  spherical particles
containing 90% of Compound B (X250)

EP 3 791 867 A1

**Description**

Technical Field

The present invention relates to a hollow particle containing a medicament, specifically, a hollow particle containing a medicament as a main component in a wall (shell) part.

Background Art

In solid pharmaceutical preparations, in general, a medicament alone is granulated, or a medicament and other formulated component are mixed and granulated to produce medicament-containing particles, which are then mixed with other components, mixed with other granules, or added with other components, further granulated and the like, and the mixture is tableted to give tablets, or formulated to give granules, or packed in a capsule to give a capsule agent.

Furthermore, to achieve medicament absorption at a desired site at a desired time, thereby to afford the desired efficacy, it is necessary to either impart the desired functions such as enteric solubility, gastric solubility and the like to the above-mentioned medicament-containing particle itself, or further apply a treatment capable of imparting the desired functions.

More effective treatments can be applied by imparting functions to the medicament-containing particle. To provide a tablet which is most popular as a solid pharmaceutical preparation, the medicament-containing particle needs to have strength sufficient to prevent breakage leading to an impairment of the function in the compression step. However, it is not easy to simultaneously solve, in a medicament-containing particle imparted with functions such as water-solubility, gastric solubility, enteric solubility and the like, enhancement of particle strength while maintaining appropriate dissolution at a desired site.

In the case of an orally disintegrating tablet, medicament-containing particles having a particle size controlled to prevent a gritty feel in the oral cavity are further necessary. In the case of a capsule, fluidity permitting encapsulation of a given amount of medicament-containing particles is necessary.

In general, moreover, a method of producing medicament-containing particles is also known, which includes coating core particles with a medicament. To reduce the size of preparation, particles having a high medicament content are required. However, the method of producing medicament-containing particles by coating core particles with a medicament is associated with problems such as a long time required for medicament coating, a large size of the obtained particles, a failure to achieve sufficiently high medicament content and the like.

To impart desired functions, for example, conventional fluidized-bed granulators (including rotating fluidized-bed granulator, Wurster-type fluidized-bed granulator and the like), hybrid fluidized-bed granulator equipped with a grinding mechanism and the like are used, and a method including coating a medicament alone, or a mixture of a medicament and other additive, with a functional additive having, for example, enteric solubility, gastric solubility and the like, and a method including coating pre-produced medicament-containing particles with a functional additive can be mentioned. However, medicament-containing particles coated by this method have problems in that they generally have a low strength and are brittle, have many concaves and convexes on the surface due to their multicore shape, and have low fluidity.

In addition, a method including mixing a medicament alone, or a mixture of a medicament and other additive, with a large amount of a functional additive such as a functional polymer and the like by a agitating granulator, adding a binder solution, and granulating the mixture can be mentioned. By this method, however, a medicament-containing particle having a desired function generally requires use of a larger amount of a functional additive, a particle having a high medicament content cannot be prepared, and obtained respective particles are not homogeneous. Since agitating granulation is generally a mechanism of granulation with compression, a medicament-containing particle having high density can be obtained. Since the density is too high, a problem in the mixing uniformity occurs thereafter when the particle is mixed with other additive and tableted. The method further has a problem that control of the particle size of medicament-containing particles is generally difficult. In addition, the method has a further problem that medicament-containing particles are disintegrated slowly, and dissolution of the medicament is delayed when a water-soluble polymer is used as a functional additive in the method.

As other method, a method including coating core particles with a medicament or a medicament-containing composition to give medicament-containing particles, and further coating them with a functional additive can be mentioned. In this method, however, the further coating step prolongs the operation time and increases the cost. In addition, the method has a problem that the particle size of the medicament-containing particles becomes large since a medicament layer and a functional additive layer are laminated on the core particles.

Patent document 1 aiming at coating for release control thereafter discloses a process for preparation of a spherical fine particle having an average particle size of not more than 200 $\mu$m, comprising adding a binder solution to a mixture of a filler powder having the property to retain a solvent and a medicament powder, and granulating the mixture by high-speed rolling. It is described that, to achieve the function of release control according to this method, coating and the like are necessary thereafter.

Patent document 2 discloses a method of producing a single-core particle. Specifically, it describes a method of producing a hollow spherical particle, including mixing a granulated substance obtained by adding dropwise an aqueous solution containing a medicament as an active ingredient and/or a binder into liquid nitrogen, with a filler and/or a powder optionally containing the medicament in a fluidized-bed granulator at a temperature not less than the ice-thawing temperature, or fluidizing granulating them while thawing to allow coexistent powder to be attached thereto. However, the description relating to the manufacturing equipment in the patent document reads, "an apparatus free of a large impact during granulation and capable of uniformly mixing a powder and a frozen granulated substance can be used. The large impact here means an impact that does not break granules." As is clear therefrom, since the described method requires a manufacturing equipment that does not break granules, the strength of the granule is problematic. In the described method, moreover, a granulated substance is produced by dropwise addition to liquid nitrogen and a powder is attached thereto. Therefore, the size depends on the size of the granulated ice substance and particles with a size of 0.5 mm - 10 mm are produced, which is larger than that of particles in general use. It is described, moreover, that only a water-soluble polymer can be used for granulation according to the method, and therefore, the function of release control can be imparted only when coating and the like are performed thereafter.

Patent document 3 discloses a medicament-containing particle comprising components such as a medicament, a water-soluble polymer, and sugar or sugar alcohol, which is obtained by granulating and particulating each component. Specifically, it discloses a particle obtained by charging a medicament, D-mannitol and polyvinylpyrrolidone in a high shear granulator, vertical granulator, and granulating the mixture while spraying purified water.

Patent document 4 discloses a constitution comprising a core and a film layer coating the core, and the core contains at least a medicament with an uncomfortable taste and a water-swelling substance. Specifically, it discloses a core (particle) obtained by charging a medicament, low-substituted hydroxypropylcellulose, lactose hydrate, and hydroxypropylcellulose in a high shear granulator, vertical granulator, and granulating the mixture while adding dropwise 95% ethanol solution.

Non-patent document 1 describes, for the purpose of selecting a disintegrant suitable for the preparation of an orally fast-disintegrating tablet, a method of preparing granules for tablet molding, which comprises mixing acetaminophen, mannitol and the disintegrant with agitating, and agitating granulating the mixture while adding an aqueous hydroxypropylcellulose solution. Specifically, it discloses a granule (particle) obtained by charging a medicament, D-mannitol and low-substituted hydroxypropylcellulose in a high shear granulator, adding dropwise a granulation liquid obtained by dissolving hydroxypropylcellulose in purified water, and granulating the mixture.

However, all of the particles produced by using the starting materials and methods specifically described in patent documents 3, 4, and non-patent document 1 are not hollow particles as shown in the below-mentioned Comparative Examples.

As mentioned above, it is not easy to satisfactorily impart strength and functionality to a medicament-containing particle simultaneously. In addition, it is not known to conveniently produce a particle having a desired particle size and good fluidity, capable of increasing a medicament content, superior in particle homogeneity, and mixing uniformity with other component.

[Document List]

[patent documents]

    patent document 1: JP-A-2000-128774
    patent document 2: JP-A-2000-72660
    patent document 3: WO2011/019043
    patent document 4: JP-A-H03-130214

[non-patent document]

non-patent document 1: Reports of the Mie Prefecture Industrial Research Institute ,2010,Vol.34,pp.30-37

## SUMMARY OF THE INVENTION

### Problems to be Solved by the Invention

A technique for conveniently obtaining a medicament-containing particle having sufficient strength durable against compression, coating and the like, and imparted with desired functionality capable of controlling dissolution at a desired site and the like has been desired. In addition, a technique for conveniently producing particles having a desired particle size and good fluidity, capable of increasing a medicament content, superior in the homogeneity of particles, and showing good mixing uniformity with other components has been desired.

### Means of Solving the Problems

The present inventors have conducted intensive studies and found that a medicament-containing particle having a sufficient particle strength, and capable of exerting polymer function such as good disintegration property of a particle itself, dissolution control at a desired site and the like can be produced efficiently by a highly convenient means including mixing a medicament powder and a polymer, particularly a polymer having desired functionality, and agitating granulating the mixture while spraying a solvent capable of dissolving the polymer, which resulted in the completion of the present invention. They have found that the particle has a hollow structure. According to the present invention, moreover, they have found that the particle size and the particle size distribution width of the medicament-containing particle can be freely controlled and particles according to the purpose can be produced conveniently. According to the present invention, furthermore, they have found that a particle having good fluidity, capable of increasing a medicament content, superior in particle homogeneity, and showing good mixing uniformity with other components can be produced.

Accordingly, the present invention relates to the following.

[1] A hollow particle, wherein the particle is composed of a shell and a hollow, and the shell comprises a medicament and a polymer, and a volume ratio of the hollow relative to the whole particle is 1% - 50% (to be also referred to as "the medicament-containing particle of the present invention" in the present specification).

[2] A hollow particle having a structure wherein a hollow is surrounded by a wall composed of a composition comprising a medicament and a polymer, and having a hollow volume ratio of 1% - 50% relative to the whole particle.

[3] A hollow particle having a hollow structure comprising a medicament and a polymer, and a hollow volume ratio of 4% - 50% relative to the whole particle.

In the present invention, the hollow particle which is a "particle composed of a shell and a hollow, wherein the shell comprises a medicament and a polymer" and the hollow particle which is a "hollow particle having a structure wherein the hollow is surrounded by a wall composed of a composition comprising a medicament and a polymer" and the hollow particle which is a "particle having a hollow structure comprising a medicament and a polymer" (or the "particle having a hollow structure comprising a medicament and a polymer") mean the same.

[4] The hollow particle of the above-mentioned [1] or [2], which has a shell thickness (or wall thickness) of not less than 15 $\mu$m.

[5] The hollow particle of any of the above-mentioned [1] - [4], wherein the hollow (or hollow structure) has a diameter of not less than 10 $\mu$m.

[6] The hollow particle of any of the above-mentioned [1] - [5], wherein the polymer used as a starting material has an average particle size of not less than 5-fold that of the medicament used as a starting material.

[7] The hollow particle of any of the above-mentioned [1] - [5], wherein the polymer used as a starting material has an average particle size of not less than 10-fold that of the medicament used as a starting material.

[8] The hollow particle of any of the above-mentioned [1] - [5], wherein the polymer used as a starting material has an average particle size of not less than 15-fold that of the medicament used as a starting material.

[9] The hollow particle of any of the above-mentioned [1] - [5], wherein the polymer used as a starting material has an average particle size of not less than 25-fold that of the medicament used as a starting material.

[10] The hollow particle of any of the above-mentioned [1] - [9], which has a medicament content of 0.1 - 96 wt% per 100 wt% of the hollow particle.

[11] The hollow particle of any of the above-mentioned [1] - [10], which has a polymer content of 4 - 50 wt% per 100 wt% of the hollow particle.

[12] The hollow particle of any of the above-mentioned [1] - [11], wherein the polymer is one or more kinds selected from the group consisting of a water-soluble polymer, a water-insoluble polymer, an enteric polymer, a gastric soluble polymer and a biodegradable polymer.

[13] The hollow particle of the above-mentioned [12], wherein the polymer includes a water-soluble polymer.

[14] The hollow particle of the above-mentioned [12], wherein the polymer is one or more kinds selected from the group consisting of a water-insoluble polymer, an enteric polymer, a gastric soluble polymer and a biodegradable polymer.

[15] The hollow particle of the above-mentioned [12] or [13], wherein the water-soluble polymer is selected from the group consisting of methylcellulose, hydroxypropylcellulose, hydroxypropylmethylcellulose, hydroxyethylcellulose, hydroxymethylcellulose, carboxymethylcellulose, polyvinylpyrrolidone, polyvinyl alcohol, copolyvidone, polyethylene glycol, polyvinyl alcohol-acrylic acid-methyl methacrylate copolymer, vinyl acetate-vinylpyrrolidone copolymer, polyvinyl alcohol-polyethylene glycol-graft copolymer, pregelatinized starch, dextrin, dextran, pullulan, alginic acid, gelatin, pectin, and a mixture of one or more kinds thereof.

[16] The hollow particle of the above-mentioned [12] or [14], wherein the water-insoluble polymer is selected from the group consisting of ethylcellulose, acetyl cellulose, aminoalkylmethacrylate copolymer RS, ethyl acrylate-methyl methacrylate copolymer dispersion, vinyl acetate resin, and a mixture of one or more kinds thereof.

[17] The hollow particle of the above-mentioned [12] or [14], wherein the enteric polymer is selected from the group consisting of hydroxypropylmethylcellulose acetate succinate, hydroxypropylmethylcellulose phthalate, methacrylic acid copolymer L, methacrylic acid copolymer LD, dried methacrylic acid copolymer LD, methacrylic acid copolymer S, methacrylic acid-acrylic acid n-butyl copolymer, and a mixture of one or more kinds thereof.

[18] The hollow particle of the above-mentioned [12] or [14], wherein the gastric soluble polymer is selected from the group consisting of polyvinylacetal diethylaminoacetate, aminoalkylmethacrylate copolymer E, and a mixture of one or more kinds thereof.

[19] The hollow particle of the above-mentioned [12] or [14], wherein the biodegradable polymer is selected from the group consisting of polylactic acid, polyglycolic acid, polycaprolactone and copolymer thereof, collagen, chitin, chitosan, and a mixture of one or more kinds thereof.

[20] The hollow particle of any of the above-mentioned [1] - [19], wherein the composition constituting the shell (or wall) further comprises other additive.

[21] The hollow particle of the above-mentioned [20], wherein said other additive is selected from the group consisting of filler, binder, sweetening agent, corrigent, smell masking agent, flavor, fluidizer, antistatic agent, colorant, disintegrant, lubricant, plasticizer, anticoagulant and coating agent.

[22] The hollow particle of the above-mentioned [20], wherein said other additive is selected from the group consisting of filler, binder, sweetening agent, corrigent, smell masking agent, flavor, fluidizer, antistatic agent, colorant and coating agent.

[23] The hollow particle of any of the above-mentioned [20] - [22], wherein the other additive used as a starting material has an average particle size of not more than 1/5 of the average particle size of the polymer used as a starting material.

[24] The hollow particle of any of the above-mentioned [20] - [22], wherein the other additive used as a starting material has an average particle size of not more than 1/10 of the average particle size of the polymer used as a starting material.

[25] The hollow particle of any of the above-mentioned [20] - [22], wherein the other additive used as a starting material has an average particle size of not more than 1/15 of the average particle size of the polymer used as a starting material.

[26] The hollow particle of any of the above-mentioned [20] - [22], wherein the other additive used as a starting material has an average particle size of not more than 1/25 of the average particle size of the polymer used as a starting material.

[27] The hollow particle of any of the above-mentioned [20] - [22], wherein a mixed powder of the medicament and the other additive used as a starting material has an average particle size of not more than 1/5 of the average particle size of the polymer used as a starting material.

[28] The hollow particle of any of the above-mentioned [20] - [22], wherein a mixed powder of the medicament and the other additive used as a starting material has an average particle size of not more than 1/10 of the average particle size of the polymer used as a starting material.

[29] The hollow particle of any of the above-mentioned [20] - [22], wherein a mixed powder of the medicament and the other additive used as a starting material has an average particle size of not more than 1/15 of the average particle size of the polymer used as a starting material.

[30] The hollow particle of any of the above-mentioned [20] - [22], wherein a mixed powder of the medicament and the other additive used as a starting material has an average particle size of not more than 1/25 of the average particle size of the polymer used as a starting material.

[31] The hollow particle of any of the above-mentioned [1] - [30], wherein the hollow particle has an aspect ratio of 1.0 - 1.5.

[32] The hollow particle of any of the above-mentioned [1] - [31], wherein the hollow particle has a particle shell strength (or particle wall strength) of not less than 2.0 MPa.

[33] The hollow particle of any of the above-mentioned [1] - [31], wherein the hollow particle has a particle shell strength (or particle wall strength) of not less than 3.0 MPa.

[34] A pharmaceutical composition comprising a plurality of the hollow particle of any of the above-mentioned [1] - [33].

[35] The pharmaceutical composition of the above-mentioned [34], wherein the hollow particle has a particle size distribution width (D90/D10) of not more than 6.

[36] The pharmaceutical composition of the above-mentioned [34] or [35], wherein the hollow particle has an average particle size of 50 - 1000 μm.

[37] The pharmaceutical composition of the above-mentioned [34] or [35], wherein the hollow particle has an average particle size of 50 - 500 μm.

[38] The pharmaceutical composition of any of the above-mentioned [34] - [37], which is in the form of any of granule, tablet and capsule.

[39] The pharmaceutical composition of the above-mentioned [38], which is in the form of a tablet.

[40] A process for preparation of the hollow particle of any of the above-mentioned [1] - [5] and [12] - [19], which comprises a step of granulating a powder mixture containing a medicament and a polymer, while spraying a solvent capable of dissolving the polymer.

[41] The process for preparation of the above-mentioned [40], wherein the granulation is agitating granulation.

[42] The process for preparation of the above-mentioned [40] or [41], wherein the polymer in the powder mixture has an average particle size of not less than 5-fold that of the medicament.

[43] The process for preparation of the above-mentioned [40] or [41], wherein the polymer in the powder mixture has an average particle size of not less than 10-fold that of the medicament.

[44] The process for preparation of the above-mentioned [40] or [41], wherein the polymer in the powder mixture has an average particle size of not less than 15-fold that of the medicament.

[45] The process for preparation of the above-mentioned [40] or [41], wherein the polymer in the powder mixture has an average particle size of not less than 25-fold that of the medicament.

[46] The process for preparation of any of the above-mentioned [40] - [45], wherein the powder mixture further contains an additive other than the medicament and the polymer.

[47] The process for preparation of the above-mentioned [46], wherein the polymer in the powder mixture has an average particle size of not less than 5-fold that of the medicament and/or other additive.

[48] The process for preparation of the above-mentioned [46], wherein the polymer in the powder mixture has an average particle size of not less than 10-fold that of the medicament and/or other additive.

[49] The process for preparation of the above-mentioned [46], wherein the polymer in the powder mixture has an average particle size of not less than 15-fold that of the medicament and/or other additive.

[50] The process for preparation of the above-mentioned [46], wherein the polymer in the powder mixture has an average particle size of not less than 25-fold that of the medicament and/or other additive.

[51] The process for preparation of the above-mentioned [46], wherein the polymer in the powder mixture has an average particle size of not less than 5-fold that of the mixed powder of the medicament and other additive.

[52] The process for preparation of the above-mentioned [46], wherein the polymer in the powder mixture has an average particle size of not less than 10-fold that of the mixed powder of the medicament and other additive.

[53] The process for preparation of the above-mentioned [46], wherein the polymer in the powder mixture has an average particle size of not less than 15-fold that of the mixed powder of the medicament and other additive.

[54] The process for preparation of the above-mentioned [46], wherein the polymer in the powder mixture has an average particle size of not less than 25-fold that of the mixed powder of the medicament and other additive.

[55] The process for preparation of any of the above-mentioned [46] - [54], wherein said other additive is selected from the group consisting of filler, binder, sweetening agent, corrigent, smell masking agent, flavor, fluidizer, antistatic agent, colorant, disintegrant, lubricant, plasticizer, anticoagulant and coating agent.

[56] The process for preparation of any of the above-mentioned [46] - [54], wherein said other additive is selected from the group consisting of filler, binder, sweetening agent, corrigent, smell masking agent, flavor, fluidizer, antistatic agent, colorant and coating agent.

[57] A process for preparation of a hollow particle, which comprises a step of granulating a powder mixture containing a medicament and a polymer, while spraying a solvent capable of dissolving the polymer.

[58] A hollow particle obtained by granulating a powder mixture containing a medicament and a polymer, while spraying a solvent capable of dissolving the polymer.

[59] The hollow particle of any of the above-mentioned [1] - [33], which is obtained by granulating a powder mixture containing a medicament and a polymer, while spraying a solvent capable of dissolving the polymer.

Effect of the Invention

Since the medicament-containing particle of the present invention has a sufficient strength, processing such as compressing, coating and the like can be performed easily. In addition, since the polymer to be added can afford desired functions (e.g., rapid disintegration property, rapid dissolution property, enteric, gastric solubility, sustained-release, bitter taste masking etc.), it can be more conveniently applied to a preparation, and a preparation that makes a medicament absorbed at a desired site at a desired time and obtains desired efficacy can be provided. Furthermore, since the particle size and the particle size distribution width of a medicament-containing particle can be freely controlled by selecting the particle size and particle size distribution of the polymer, a particle suitable for the purpose can be produced easily.

Since the medicament-containing particle of the present invention permits preparation of a particle containing a high content of a medicament by increasing the medicament ratio, the size of the preparation can be reduced, and compliance of a preparation can be improved. According to the present invention, a medicament-containing particle having high sphericity can be produced, and the medicament-containing particle of the present invention having high sphericity improves bad fluidity of a medicament, even a small amount thereof at any particle size and any particle size distribution width can be filled in a capsule highly accurately, and superior in the particle homogeneity. Therefore, even when a small amount is filled in a capsule, quality inconsistency for each capsule does not occur easily and, when preparations having varying doses are provided in the early clinical development of a pharmaceutical product, those having any doses can be supplied conveniently.

Moreover, since the particle of the present invention has a hollow, the transfer rate of the particle in the gastrointestinal tract can be varied by changing the hollow ratio. Furthermore, since the particle density can be appropriately controlled, the mixing uniformity becomes fine when the particles of the present invention and other additives are mixed and the mixture is tableted.

Brief Description of the Drawings

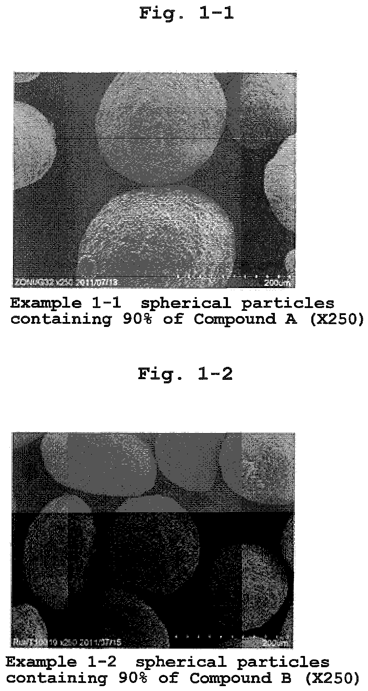

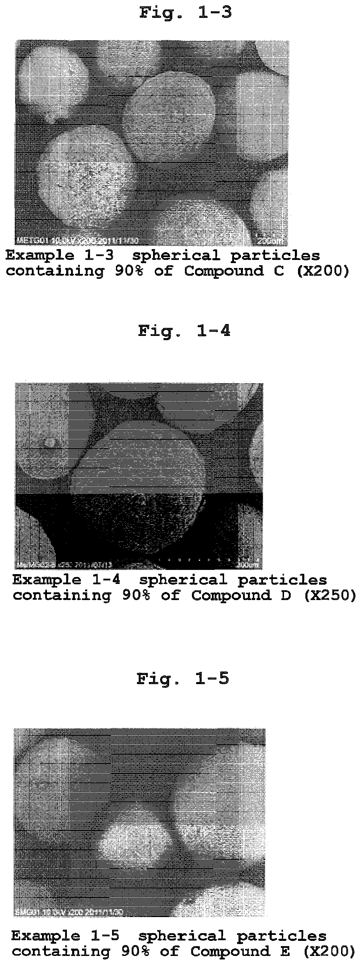

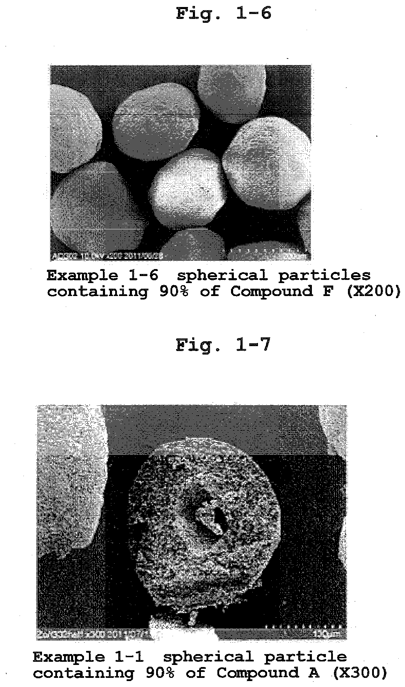

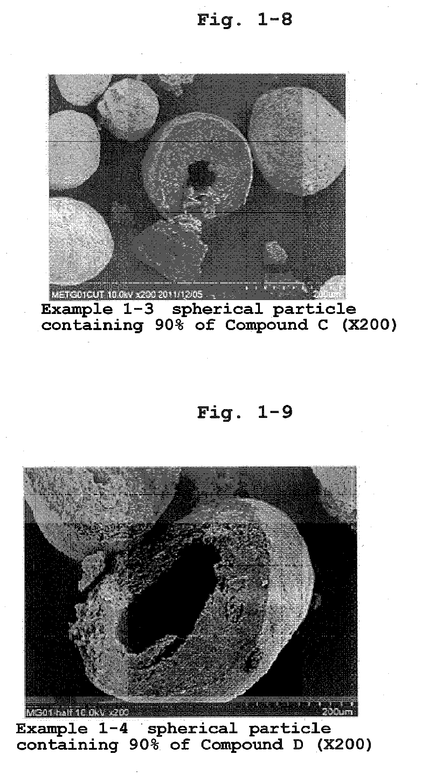

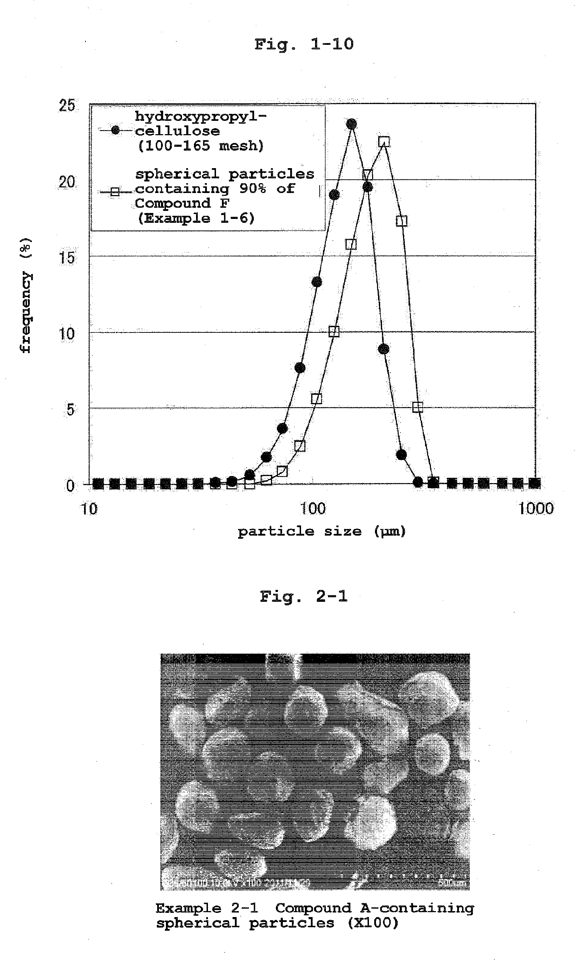

[Fig. 1-1]Fig. 1-1 is an electron micrograph showing the appearance of spherical particles containing 90% of Compound A of Example 1-1.
[Fig. 1-2]Fig. 1-2 is an electron micrograph showing the appearance of spherical particles containing 90% of Compound B of Example 1-2.
[Fig. 1-3]Fig. 1-3 is an electron micrograph showing the appearance of spherical particles containing 90% of Compound C of Example 1-3.
[Fig. 1-4]Fig. 1-4 is an electron micrograph showing the appearance of spherical particles containing 90% of Compound D of Example 1-4.
[Fig. 1-5]Fig. 1-5 is an electron micrograph showing the appearance of spherical particles containing 90% of Compound E of Example 1-5.
[Fig. 1-6]Fig. 1-6 is an electron micrograph showing the appearance of spherical particles containing 90% of Compound F of Example 1-6.
[Fig. 1-7]Fig. 1-7 is an electron micrograph showing the cross-section of spherical particle containing 90% of Compound A of Example 1-1.
[Fig. 1-8]Fig. 1-8 is an electron micrograph showing the cross-section of spherical particle containing 90% of Compound C of Example 1-3.
[Fig. 1-9]Fig. 1-9 is an electron micrograph showing the cross-section of spherical particle containing 90% of Compound Compound D of Example 1-4.
[Fig. 1-10]Fig. 1-10 shows the relationship between the particle size distribution of a polymer (hydroxypropylcellulose) and the particle size distribution of medicament-containing particles in Example 1-6.
[Fig. 2-1]Fig. 2-1 is an electron micrograph showing the appearance of Compound A-containing spherical particles of Example 2-1.
[Fig. 2-2]Fig. 2-2 is an electron micrograph showing the appearance of Compound A-containing spherical particles of Example 2-2.

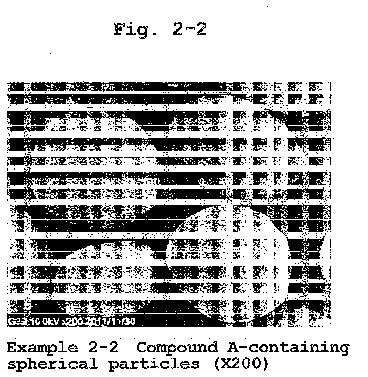

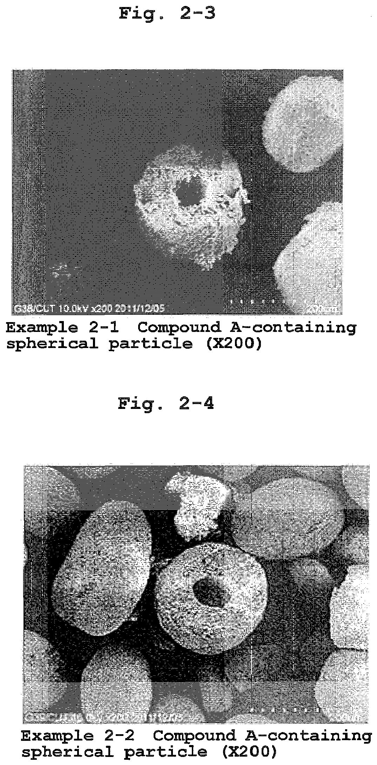

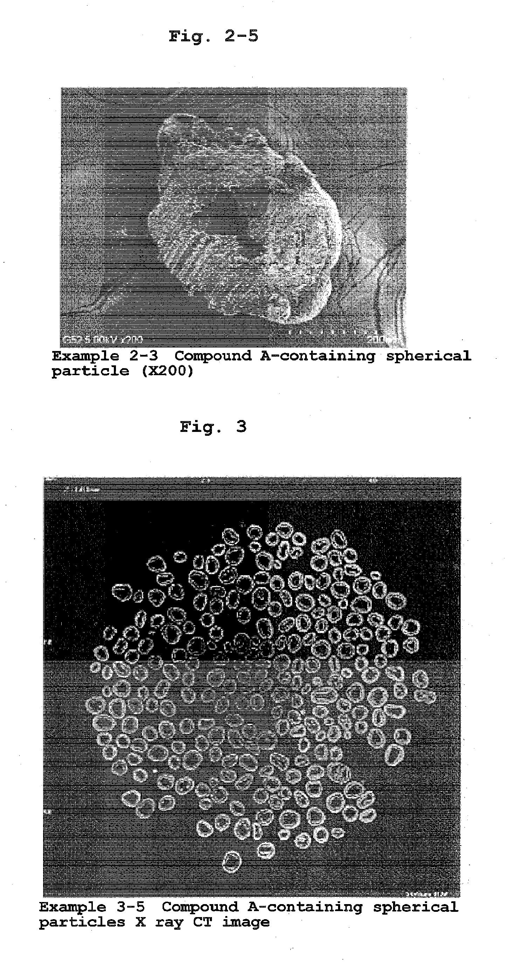

[Fig. 2-3]Fig. 2-3 is an electron micrograph showing the cross-section of Compound A-containing spherical particle of Example 2-1.
[Fig. 2-4]Fig. 2-4 is an electron micrograph showing the cross-section of Compound A-containing spherical particle of Example 2-2.
[Fig. 2-5]Fig. 2-5 is an electron micrograph showing the cross-section of Compound A-containing spherical particle of Example 2-3.
[Fig. 3]Fig. 3 is an X ray CT image of Compound A-containing spherical particles of Example 3-5.

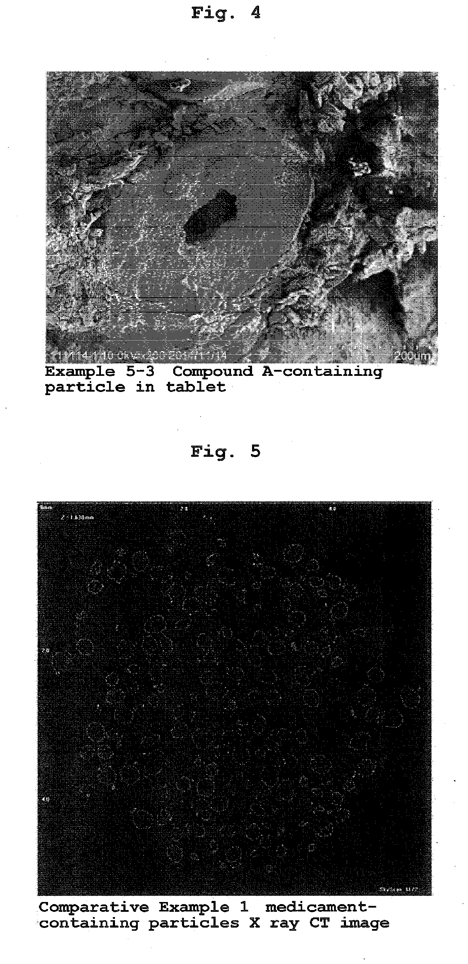

[Fig. 4]Fig. 4 is an electron micrograph showing the cross-section of Compound A-containing particle in a tablet of Example 5-3.
[Fig. 5]Fig. 5 is an X ray CT image of medicament-containing particles of Comparative Example 1.

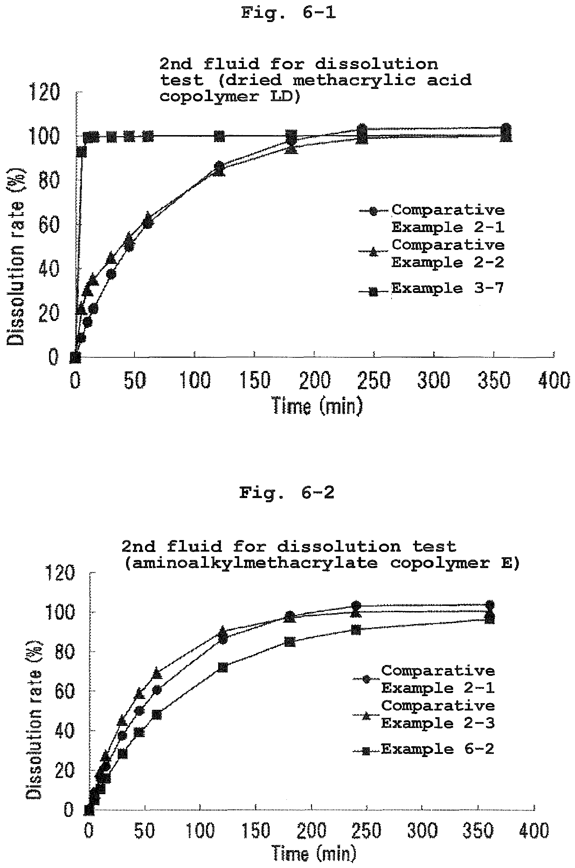

[Fig. 6-1]Fig. 6-1 shows dissolution rates of Compound A-containing spherical particles in 2nd fluid for dissolution test in Comparative Examples 2-1, 2-2, and Example 3-7 using dried methacrylic acid copolymer LD as the polymer.
[Fig. 6-2]Fig. 6-2 shows dissolution rates of Compound A-containing spherical particles in 2nd fluid for dissolution test in Comparative Examples 2-1, 2-3, and Example 6-2 using aminoalkylmethacrylate copolymer E as the polymer.

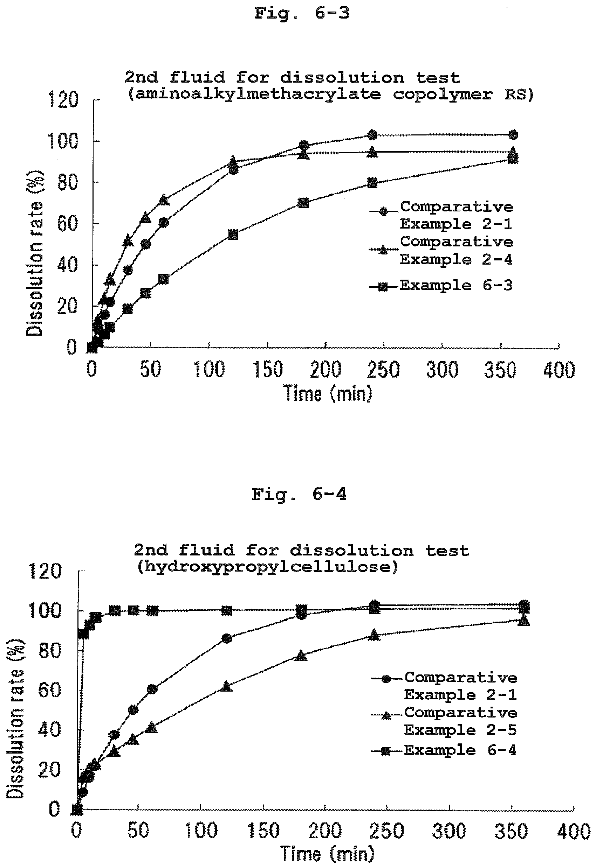

[Fig. 6-3]Fig. 6-3 shows dissolution rates of Compound A-containing spherical particles in 2nd fluid for dissolution test in Comparative Examples 2-1, 2-4, and Example 6-3 using aminoalkylmethacrylate copolymer RS as the polymer.
[Fig. 6-4]Fig. 6-4 shows dissolution rates of Compound A-containing spherical particles in 2nd fluid for dissolution test in Comparative Examples 2-1, 2-5, and Example 6-4 using hydroxypropylcellulose as the polymer.

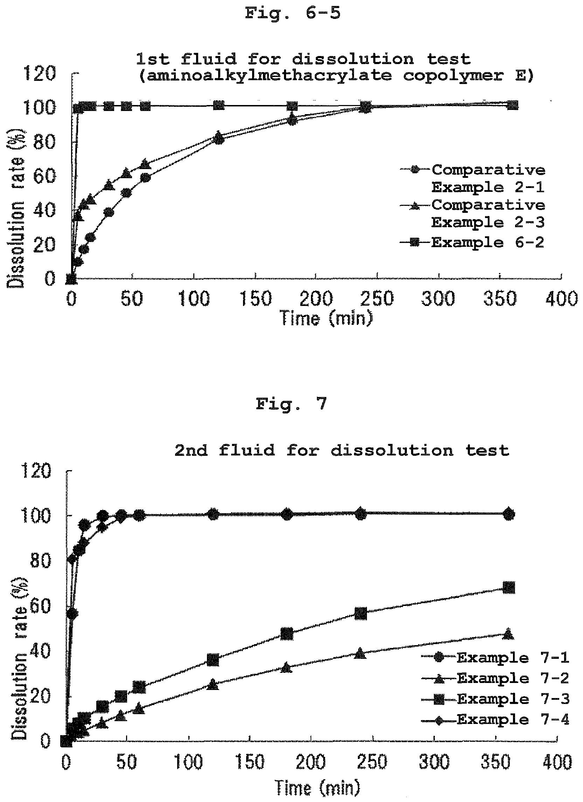

[Fig. 6-5]Fig. 6-5 shows dissolution rates of Compound A-containing spherical particles in 2nd fluid for dissolution test in Comparative Examples 2-1, 2-3, and Example 6-2 using aminoalkylmethacrylate copolymer E as the polymer.
[Fig. 7]Fig. 7 shows dissolution rates of Compound G-containing spherical particles in 2nd fluid for dissolution test in Example 7-1, 7-2, 7-3, 7-4 using various polymers.

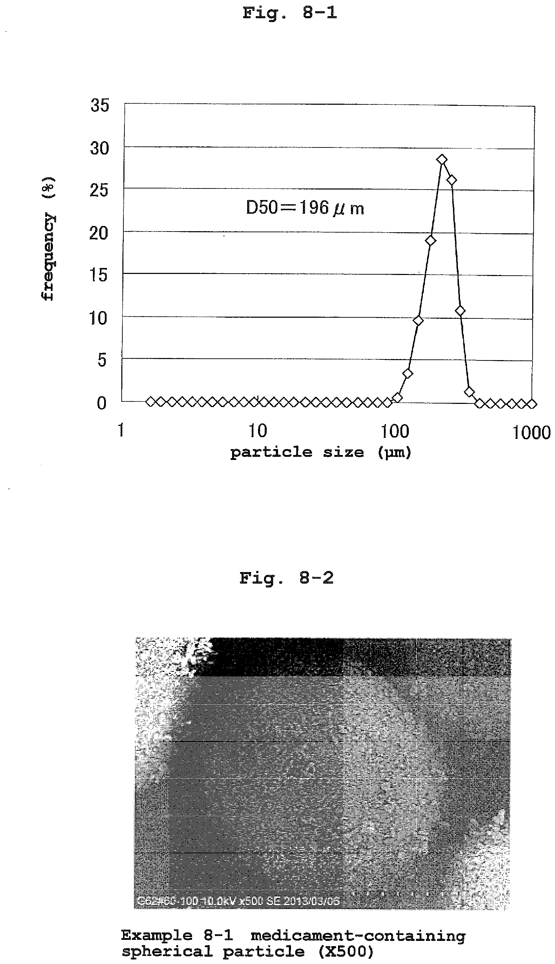

[Fig. 8-1]Fig. 8-1 shows the particle size distribution of Compound A-containing spherical particles of Example 8-1.
[Fig. 8-2]Fig. 8-2 is an electron micrograph showing the appearance of Compound A-containing spherical particle of Example 8-1.

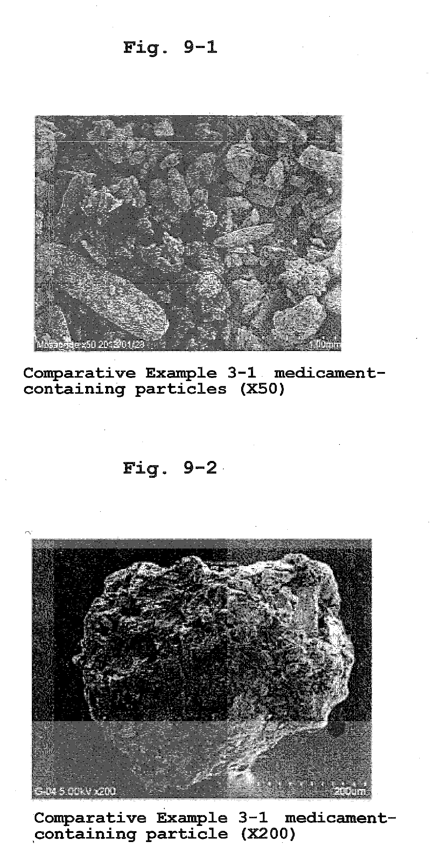

[Fig. 9-1]Fig. 9-1 is an electron micrograph showing the appearance of medicament-containing particles of Comparative Example 3-1.
[Fig. 9-2]Fig. 9-2 is an electron micrograph showing the cross-section of Compound A-containing particle of Comparative Example 3-1.

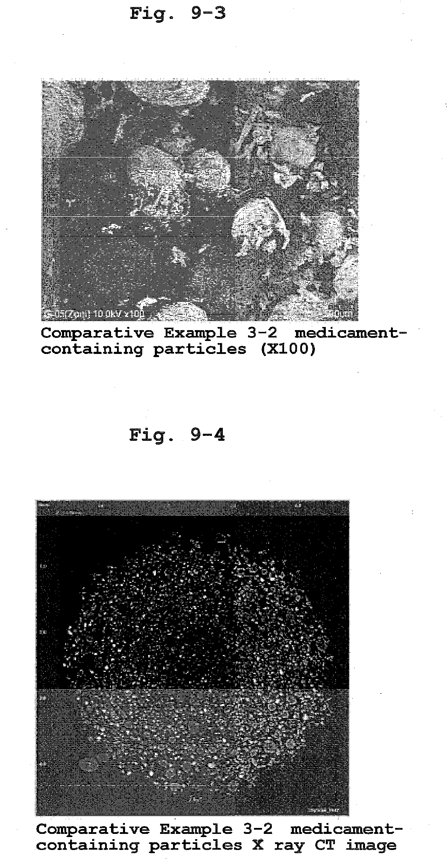

[Fig. 9-3]Fig. 9-3 is an electron micrograph showing the appearance of Compound A-containing particles of Comparative Example 3-2.
[Fig. 9-4]Fig. 9-4 is an X ray CT image of Compound A-containing particles of Comparative Example 3-2.

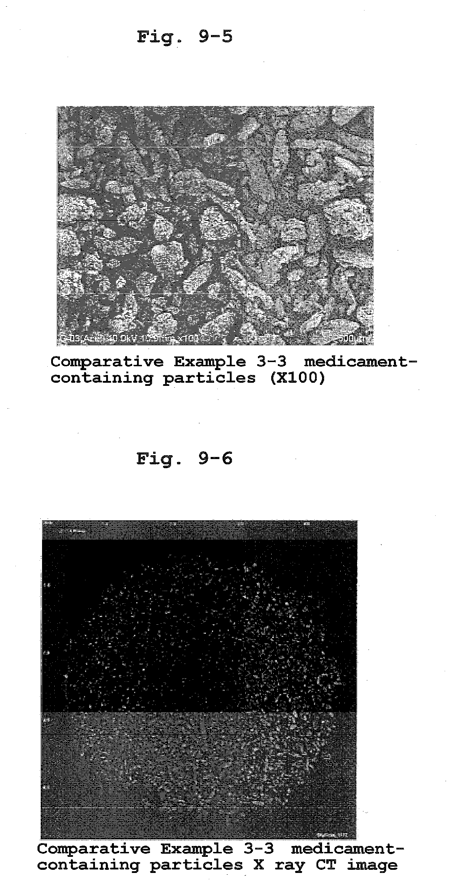

[Fig. 9-5]Fig. 9-5 is an electron micrograph showing the appearance of medicament-containing particles of Comparative Example 3-3.
[Fig. 9-6]Fig. 9-6 is an X ray CT image of medicament-containing particles of Comparative Example 3-3.

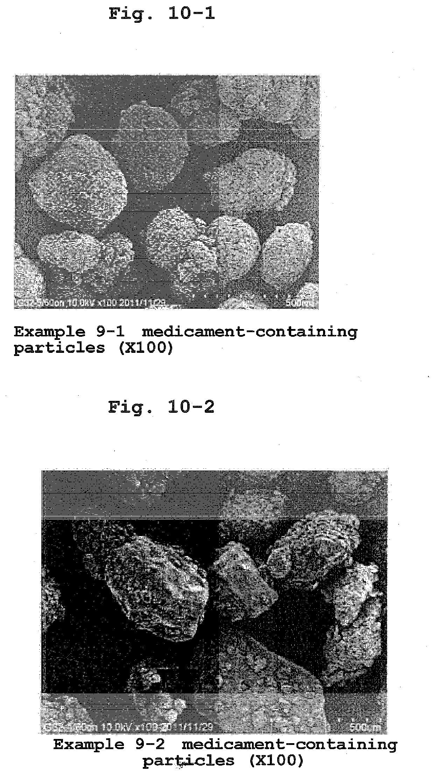

[Fig. 10-1]Fig. 10-1 is an electron micrograph showing the appearance of Compound A-containing particles of Example 9-1.
[Fig. 10-2]Fig. 10-2 is an electron micrograph showing the appearance of Compound A-containing particles of Example 9-2.

Description of Embodiments

The present invention is explained in more detail in the following.
The medicament-containing particle of the present invention contains a medicament and a polymer as essential constituent elements. The particle means both one particle and an aggregate of a plurality of particles.
In the present invention, the "average particle size" means cumulative 50% particle size D50 in the volume based measurement of powder particles. Such average particle size is measured by a laser diffraction particle size analyzer (e.g., Particle Viewer manufactured by POWREX CORPORATION, or SALD-3000J manufactured by Shimadzu Corporation, or HELOS&RODOS manufactured by Sympatec GmbH) by volume basis.

(i) Medicament

Medicaments can be used without a particular limitation. The "medicament" to be used for the method of the present invention may be any medicament or compound irrespective of properties such as basic, acidic, ampholytic, neutral and the like, and solubility. Among those, from the aspects of stability and easy handling, a crystalline medicament or compound is preferable. In addition, a mixture of one or more kinds of medicaments may be used. The particle of the present invention is also effective for medicaments having low solubility. For example, when the following water-soluble polymer is used as the polymer, rapid disintegration property and rapid dissolution property can be exhibited.
A smaller average particle size of a medicament used as a starting material in the present invention can afford a medicament-containing particle having a smoother surface. It is preferably not more than 20 $\mu$m, more preferably not more than 10 $\mu$m, further preferably not more than 5 $\mu$m, most preferably not more than 3 $\mu$m. The average particle size of a medicament is generally not less than 0.1 $\mu$m.
In the present invention, the average particle size of a medicament may be any as long as it is within the above-mentioned range as a starting material, and may vary depending on the preparation process of the medicament-containing particles and the like.
Where necessary, the medicament may be pulverized to have a desired particle size before preparation of particles. While the pulverization is performed by a conventional method such as pulverization using a fine grinding mill and the like, very fine particles (average particle size not more than 1 $\mu$m) may be produced. While the medicament content can be set freely, a preferable amount of the medicament to be used is not more than 96 wt%, preferably not more than 94 wt%, more preferably not more than 92 wt%, further preferably not more than 90 wt%, per 100 wt% of the medicament-containing particles (hollow particles) to be prepared. Specifically, it is 0.1 - 96 wt%, preferably 0.1 - 95.9 wt%, more preferably 1 - 94 wt%, further preferably 5 - 92 wt%, most preferably 10 - 90 wt%, per 100 wt% of the medicament-containing particles.

In the present invention, it is also possible to produce not only particles containing a medicament at a low content but also at a high content (e.g., 50 - 96 wt%, preferably 70 - 96 wt%, more preferably 90 - 96 wt%, per 100 wt% of the medicament-containing particles). When contained at a low content, the particles can be produced by mixing other additives, preferably, additives insoluble in solvents, which are described below.

(ii) Polymer

In the present invention, the "polymer" refers to a molecule having a large relative morecular mass, and a structure composed of multiple repeats of a morecular having a small relative molecule mass, and particularly refers to a functional polymer. The aforementioned "molecule having a large relative molecular mass" has an average molecular weight (weight average molecular weight) of generally not less than 1000, preferably not less than 5000, more preferably not less than 10000. While the upper limit of the molecular weight is not particularly defined, it is preferably not more than 10000000, more preferably not more than 5000000, further preferably not more than 2000000, particularly preferably not more than 1000000. Examples of the functional polymer include water-soluble polymer, water-insoluble polymer, enteric polymer, gastric soluble polymer, and biodegradable polymer used for colon-targeting such as chitosan and the like. Preferred are water-soluble polymer, water-insoluble polymer, enteric polymer, and gastric soluble polymer. A mixture of one or more kinds of polymers may be used.

Examples of the water-soluble polymer include cellulose derivatives such as methylcellulose (e.g., trade name: SM-4, SM-15, SM-25, SM-100, SM-400, SM-1500, SM-4000, 60SH-50, 60SH-4000, 60SH-10000, 65SH-50, 65SH-400, 65SH-4000, 90SH-100SR, 90SH-4000SR, 90SH-15000SR, 90SH-100000SR), hydroxypropylcellulose (e.g., trade name: HPC-SSL, HPC-SL, HPC-L, HPC-M, HPC-H), hydroxypropylmethylcellulose (e.g., trade name: TC5-E, TC5-M, TC5-R, TC5-S, SB-4), hydroxyethylcellulose (e.g., trade name: SP200, SP400, SP500, SP600, SP850, SP900, EP850, SE400, SE500, SE600, SE850, SE900, EE820), hydroxymethylcellulose, carboxymethylcellulose (e.g., trade name: NS-300) and the like, and salts thereof, water-soluble vinyl derivatives such as polyvinylpyrrolidone (e.g., trade name: Plasdone K12, Plasdone K17, Plasdone K25, Plasdone K29-32, Plasdone K90, Plasdone K90D), polyvinyl alcohol (e.g., trade name: Gohsenol EG-05, Gohsenol EG-40, Gohsenol EG-05P, Gohsenol EG-05PW, Gohsenol EG-30P, Gohsenol EG-30PW, Gohsenol EG-40P, Gohsenol EG-40PW), Copolyvidone (e.g., trade name: Kollidon VA64, Plasdone S-630), polyethylene glycol, polyvinyl alcohol-acrylic acid-methyl methacrylate copolymer (e.g., trade name: POVACOAT), vinyl acetate-vinylpyrrolidone copolymer (e.g., trade name: Kollidon VA64), polyvinyl alcohol-polyethylene glycol-graft copolymer (e.g., trade name: Kollicoat IR) and the like, pregelatinized starch (e.g., trade name: AMICOL C), dextrin, dextran, pullulan, alginic acid, gelatin, pectin and the like. A mixture of one or more kinds of water-soluble polymers may be used. Preferred are hydroxypropylcellulose, hydroxypropylmethylcellulose, polyvinylpyrrolidone, polyvinyl alcohol and pregelatinized starch, and more preferred is hydroxypropylcellulose.

Examples of the water-insoluble polymer include water insoluble cellulose ether such as ethylcellulose (e.g., trade name: ETHOCEL (ETHOCEL 10P)), acetyl cellulose and the like, water insoluble acrylic acid copolymers such as aminoalkylmethacrylate copolymer RS (e.g., trade name: Eudragit RL100, Eudragit RLPO, Eudragit RL30D, Eudragit RS100, Eudragit RSPO, Eudragit RS30D), ethyl acrylate-methyl methacrylate copolymer dispersion (e.g., trade name: Eudragit NE30D) and the like, vinyl acetate resin and the like. A mixture of one or more kinds of water-insoluble polymers may be used. Preferred are ethylcellulose and aminoalkylmethacrylate copolymer RS. In the present invention, sustained-release and the function of a bitter taste masking for a medicament having a bitter taste can be imparted by using a water-insoluble polymer as the polymer.

Examples of the enteric polymer include hydroxypropylmethylcellulose acetate succinate (e.g., trade name: AQOAT LF, AQOAT MF, AQOAT HF, AQOAT LG, AQOAT MG, AQOAT HG), hydroxypropylmethylcellulose phthalate (e.g., trade name: HPMCP50, HPMCP55, HPMCP55S), methacrylic acid copolymers such as methacrylic acid copolymer L (e.g., trade name: Eudragit L100), methacrylic acid copolymer LD (e.g., trade name: Eudragit L30D-55), dried methacrylic acid copolymer LD (e.g., trade name: Eudragit L100-55), methacrylic acid copolymer S (e.g., trade name: Eudragit S100), methacrylic acid-acrylic acid n-butyl copolymer and the like, and the like. A mixture of one or more kinds of enteric polymers may be used. Preferred are methacrylic acid copolymer L, and dried methacrylic acid copolymer LD.

Examples of the gastric soluble polymer include gastric soluble polyvinyl derivatives such as polyvinyl acetal diethylaminoacetate and the like, gastric soluble acrylic acid copolymers such as aminoalkylmethacrylate copolymer E (e.g., trade name: Eudragit E100, Eudragit EPO) and the like, and the like. A mixture of one or more kinds of gastric soluble polymers may be used. Preferred is aminoalkylmethacrylate copolymer E.

Biodegradable polymer is a polymer decomposable in vivo. Examples thereof include polylactic acid, polyglycolic acid, polycaprolactone and copolymers thereof, collagen, chitin, chitosan (e.g., trade name: FLONAC C-100M) and the like. A mixture of one or more kinds of biodegradable polymers may be used. Preferred are polylactic acid, polyglycolic acid, polycaprolactone and copolymers thereof, gelatin, collagen, chitin, and chitosan.

In the present invention, polymer can be selected depending on the purpose. For example, to achieve rapid dissolution of medicament from a medicament particle in the gastrointestinal tract, a water-soluble polymer is preferably used as the polymer; to achieve sustained-release of a medicament, a water-insoluble polymer is preferably used as the polymer; to achieve a bitter taste masking, a water-insoluble polymer, an enteric polymer, a gastric soluble polymer and the like are preferably used; to suppress dissolution of medicament in the stomach, and facilitate dissolution in the small intestine, an enteric polymer is preferably used; and to achieve colon-targeting, chitosan and the like are preferably used. Depending on the purpose, a mixture of two or more kinds of polymers having different functions such as a water-soluble polymer, a water-insoluble polymer and the like may be used.

In the present invention, a polymer in a powdery condition is preferably used, and a polymer having a suitable average particle size and suitable particle size distribution can be selected depending on the desired average particle size and particle size distribution of the medicament-containing particle. While the examples given above also include polymers in a dispersion state, they can be used as a powder for the present invention by, for example, powderizing them by spray drying and the like. For example, to obtain a medicament-containing particle having a narrow particle size distribution, a polymer powder having a narrow particle size distribution is preferably used. To obtain a medicament-containing particle having a large average particle size, a polymer powder having a large average particle size is preferably used; and to obtain a medicament-containing particle having a small average particle size, a polymer powder having a small average particle size is preferably used. This in turn means that a medicament-containing particle having a particle size distribution suitable for the purpose can be produced by adjusting the size and particle size distribution of the polymer powder.

A preferable average particle size of a polymer used as a starting material in the present invention is not less than 0.5 $\mu$m, preferably not less than 5 $\mu$m, preferable embodiments are not less than 20 $\mu$m, not less than 25 $\mu$m, not less than 40 $\mu$m, not less than 50 $\mu$m, and a polymer having an average particle size which is not less than 5-fold, preferably not less than 10-fold, more preferably not less than 15-fold, further preferably not less than 20-fold, particularly preferably not less than 25-fold, that of a medicament used as a starting material and/or other additives described below is preferable.

In the present specification, the number of folds of the average particle size of a polymer used as a starting material relative to that of a medicament used as a starting material and/or other additive described below is shown by a particle size distribution ratio (D50/D50) of the average particle size of a polymer used as a starting material and that of a medicament used as a starting material and/or other additive described below.

For example, in the below-mentioned Example 1-1, D50 of a polymer used as a starting material (hydroxypropylcellulose (100-165 mesh)) is 137.8, D50 of a medicament used as a starting material (Compound A) is 2.7 (Table 4), the particle size distribution ratio (D50/D50) is 51.0 (137.8/2.7=51.0) (Table 38-1), and the average particle size of the polymer used as a starting material is 51.0-fold that of the medicament used as a starting material.

From the aspect of particle shell strength, a larger particle size distribution ratio (D50/D50) of a polymer used as a starting material and a mixed powder of a medicament used as a starting material and other additive is more preferable. It is generally not more than 1000-fold, preferably not more than 500-fold, more preferably not more than 100-fold. The average particle size of a polymer is generally not more than 5 mm, preferably not more than 1 mm, more preferably not more than 300 $\mu$m, further preferably not more than 250 $\mu$m, particularly preferably not more than 200 $\mu$m. A polymer powder of a certain particle size fraction can also be used selectively by, for example, a sieving method.

For example, a polymer having a desired particle size distribution can be obtained and used by appropriately selecting a sieve of a sieve number described in USP (United States Pharmacopeia), EP (European Pharmacopoeia), JP (the Japanese Pharmacopoeia), and fractionating a polymer powder. From the aspect of particle shell strength, a smaller particle size distribution width (D90/D10) of a polymer used as a starting material is more preferable.
in the present invention, the average particle size of a polymer only needs to be within the above-mentioned range as a starting material, and may vary in the preparation process and the like of a medicament-containing particle.

That is, the present invention is characterized in that a polymer is not used as a granulation liquid in the state of a solution or suspension, but mixed in a powdery condition with a medicament and then granulated while adding, for example, spraying a solvent. As long as the effect of the invention can be exhibited, a part of the polymer or medicament may be used by dissolving or suspending in a solvent. While the amount of the polymer to be used varies depending on the amounts of the medicament and other additive, particle size, the strength of the binding force of the polymer and the like, it is generally used within the range of 4 - 50 wt%, preferably 4 - 40 wt%, more preferably 6 - 40 wt% or 8 - 40 wt%, further preferably 10 - 40 wt%, still more preferably 10 - 30 wt%, particularly preferably 10 - 20 wt%, per 100 wt% of the medicament-containing particles (hollow particles) to be prepared.

From another aspect, it is used within the range of preferably 5 - 50 wt%, more preferably 5 - 40 wt%, further preferably 5 - 30 wt%, particularly preferably 5 - 25 wt%.

As the medicament-containing particle of the present invention, a particle containing 60 - 96 wt% of a medicament and 4 - 40 wt% of a polymer; preferably, a particle containing 70 - 95 wt% of a medicament and 5 - 30 wt% of a polymer; more preferably, a particle containing 80 - 90 wt% of a medicament and 10 - 20 wt% of a polymer, each per 100 wt% of the medicament-containing particle can be mentioned.

As the medicament-containing particle of the present invention, a particle containing 55 - 95.9 wt% of a medicament, 4 - 40 wt% of a polymer, and 0.1 - 5 wt% of the below-mentioned other additive; preferably, a particle containing 65 - 94.9 wt% of a medicament, 5 - 30 wt% of a polymer, and 0.1 - 5 wt% of the below-mentioned other additive; and a particle containing 75 - 89.9 wt% of a medicament, 10 - 20 wt% of a polymer, and 0.1 - 5 wt% of the below-mentioned other additive, each per 100 wt% of the medicament-containing particle can be mentioned.

As the medicament-containing particle of the present invention, a particle containing 0.1 - 95.9 wt% of a medicament, 4 - 40 wt% of a polymer, and 0.1 - 95.9 wt% of the below-mentioned other additive; preferably, a particle containing 1 - 94 wt% of a medicament, 5 - 30 wt% of a polymer, and 1 - 94 wt% of the below-mentioned other additive; and a particle containing 10 - 80 wt% of a medicament, 10 - 20 wt% of a polymer, and 10 - 80 wt% of the below-mentioned other additive, each per 100 wt% of the medicament-containing particle can be mentioned.

As the medicament-containing particle of the present invention, a particle containing 60 - 96 wt% of a medicament and 4 - 40 wt% of a polymer (preferably, a particle containing 70 - 95 wt% of a medicament and 5 - 30 wt% of a polymer; more preferably, a particle containing 80 - 90 wt% of a medicament and 10 - 20 wt% of a polymer), each per 100 wt% of the medicament-containing particle, wherein a preferable average particle size of the polymer used as a starting material is not less than 10-fold (preferably not less than 15-fold, more preferably not less than 25-fold) that of the medicament used as a starting material can be mentioned.

As the medicament-containing particle of the present invention, a particle containing 55 - 95.9 wt% of a medicament, 4 - 40 wt% of a polymer, and 0.1 - 5 wt% of the below-mentioned other additive (preferably, a particle containing 65 - 94.9 wt% of a medicament, 5 - 30 wt% of a polymer, and 0.1 - 5 wt% of the below-mentioned other additive; and more preferably a particle containing 75 - 89.9 wt% of a medicament, 10 - 20 wt% of a polymer, and 0.1 - 5 wt% of the below-mentioned other additive, each per 100 wt% of the medicament-containing particle, wherein a preferable average particle size of the polymer used as a starting material is not less than 10-fold (preferably not less than 15-fold, more preferably not less than 25-fold) that of a mixed powder of the medicament used as a starting material and the other additive can be mentioned.

As the medicament-containing particle of the present invention, a particle containing 0.1 - 95.9 wt% of a medicament, 4 - 40 wt% of a polymer, and 0.1 - 95.9 wt% of the below-mentioned other additive (preferably, a particle containing 1 - 94 wt% of a medicament, 5 - 30 wt% of a polymer, and 1 - 94 wt% of the below-mentioned other additive; and more preferably a particle containing 10 - 80 wt% of a medicament, 10 - 20 wt% of a polymer, and 10 - 80 wt% of the below-mentioned other additive, each per 100 wt% of the medicament-containing particle, wherein a preferable average particle size of the polymer used as a starting material is not less than 10-fold (preferably not less than 15-fold, more preferably not less than 25-fold) that of a mixed powder of the medicament used as a starting material and the other additive can be mentioned.

Process for preparation

The medicament-containing particle of the present invention can be produced by granulating a powder mixture containing a medicament (the above-mentioned (i)) and a polymer (the above-mentioned (ii)) while adding, for example, spraying a solvent capable of dissolving the polymer, and drying the granules.

As the granulation method, any can be selected as appropriate as long as it has an agitating function. For example, agitating granulation method, mixing agitating granulation, high shear granulation, high shear mixing agitating granulation, tumbling agitating fluidized bed granulation, and tumbling granulation can be used for the preparation. Of these, agitating granulation, mixing agitating granulation, high shear granulation, and high shear mixing agitating granulation are preferably used. Examples of the granulator used for agitating granulation, mixing agitating granulation and the like include universal mixer (manufactured by Shinagawa Machinery Works Co., Ltd.), super mixer (manufactured by KAWATA MFG Co., Ltd.), FM mixer (manufactured by NIPPON COKE & ENGINEERING. CO., LTD.), SPG series (manufactured by Fuji Paudal co., Ltd.), vertical granulator (e.g., FM-VG-05, FM-VG-100, manufactured by POWREX CORP.), high sheat mixing agitating gramulation Pharma matrix (manufactured by Nara Machinery Co., Ltd.), high speed mixer (manufactured by Fukae Powtec Corporation), GRANUMEIST (manufactured by Freund Corporation), New-Gra Machine (manufactured by Seishin Enterprise Co., Ltd.), triple master (manufactured by Shinagawa Machinery Works Co., Ltd.) and the like. In the present invention, simple fluidized bed granulation method is not preferable since drying efficiency is too high and granulation does not proceed.

As the drying method, a method known per se can be appropriately selected. For example, drying by shelf dryer or fluidized bed and the like can be mentioned and, from the aspects of productivity, drying by fluidized bed is preferable.

The "solvent" in the present invention means any solvent acceptable in the fields of pharmaceutical product, quasi-medicament, cosmetic, food and the like, and may be any as long as it can dissolve a polymer to be used. Since the medicament-containing particle of the present invention is used as a medicament, a pharmaceutically acceptable solvent is preferable. Such solvent is appropriately selected depending on the kind of the medicament, polymer and additive, and the like, and a mixture of several kinds of solvents may be used.

Examples of the "solvent" in the present invention include water, alcohol solvents (e.g., optionally substituted lower alkanol such as methanol, ethanol, n-propylalcohol, isopropylalcohol, 2-methoxyethanol, 2-ethoxyethanol and the like), ketone solvents (e.g., lower alkylketone such as acetone, methylethylketone and the like), ester solvents (e.g., lower alkyl ester of acetic acid such as ethyl acetate and the like) and a mixed solvent thereof.

Specifically, when a water-soluble polymer is used as the polymer in the present invention, a solvent capable of dissolving the polymer (e.g., water, water-containing alcohol solvent etc.) can be used as the solvent, and water or water-containing ethanol can be particularly preferably used. When a water-insoluble polymer is used as the polymer, a solvent capable of dissolving the polymer (e.g., alcohol solvent, ketone solvents, ester solvent etc.) can be used as the solvent, and solvents capable of dissolving polymers such as gastric soluble polymer, enteric polymer, chitosan and the like (e.g., alcoholic solvent, more specifically ethanol) can be used as the solvent.

While the amount of the solvent to be used in the present invention varies depending on the kind, amount and the like of the medicament and polymer, it is generally 5 - 60 parts by weight, preferably 10 - 53 parts by weight, more preferably 10 - 40 parts by weight, further preferably 15 - 40 parts by weight, per 100 parts by weight of the total amount of the components constituting the particle. It is preferably added to a powder mixture containing the medicament and the polymer by spraying.

In the present invention, a solvent is sprayed by using a Spray Gun generally used for granulation. Specific examples include Needle Spray Gun (manufactured by Tomita engineering Co., Ltd.) and the like. To increase the yield of the granule, spraying on the space other than the powder in a granulation container, namely, inner wall etc. of the granulation container, should be as little as possible and it is preferable to spray a solvent on the widest possible area of the powder in the granulation container.

For the preparation of the medicament-containing particle of the present invention, other additive may also be contained as necessary. The amount of addition thereof can be appropriately adjusted according to the kind and amount of the medicament, polymer and solvent. Other additive can be added to a mixture of the medicament and the polymer before addition of a solvent.

Other additive is preferably a powder. When the additive is a powder, the average particle size of the powder additive to be used as a starting material is not more than 20 μm, preferably not more than 10 μm, more preferably not more than 5 μm, further preferably not more than 3 μm, and an average particle size of the same level as or not more than that of the aforementioned medicament powder to be used as a starting material is preferable. When the particle size of the additive is large, a desired particle containing a polymer, a medicament and an additive cannot be formed and, when the additive is coarse, it is separated from the medicament-containing particle of the present invention. The amount of the additive to be used is not particularly limited, and a smaller amount of the additive produces a particle having a high medicament content. A particle with a low medicament content can be produced by increasing the amount of the additive. It is also possible to add the additive by dissolving or dispersing same in a solvent. When it is dissolved, the average particle size thereof is not particularly limited. When it is dispersed, the average particle size thereof is preferably of the same level as or not more than that of the aforementioned additive powder. The average particle size of other additive is generally not less than 0.005 μm.

In the present invention, the average particle size of other additive only needs to be within the above-mentioned range as the starting material, and may vary in the preparation process etc. of the medicament-containing particle.

The amount of other additive in the medicament-containing particle of the present invention can be set by decreasing the amount of the medicament, and the content of the medicament and other additive in combination can be freely set. A preferable amount of use is not more than 96 wt%, preferably not more than 94 wt%, more preferably not more than 92 wt%, further preferably not more than 90 wt%, per 100 wt% of the medicament-containing particle to be prepared. Specifically, it is 50 - 96 wt%, preferably 60 - 94 wt%, more preferably 60 - 90 wt%, further preferably 70 - 90 wt%, particularly preferably 80 - 90 wt%, per 100 wt% of the particle.

The amount of other additive in the medicament-containing particle of the present invention can be set freely. A preferable amount of use is not more than 95.9 wt%, preferably not more than 94 wt%, more preferably not more than 92 wt%, further preferably not more than 90 wt%, per 100 wt% of the medicament-containing particle to be prepared. Specifically, it is 0.1 - 95.9 wt%, preferably 1 - 94 wt%, more preferably 5 - 92 wt%, further preferably 10 - 80 wt%, per 100 wt% of the particle.

The additive is not particularly limited as long as it is generally used and, for example, filler (e.g., starch such as rice starch and the like, D-mannitol, magnesium carbonate), binder, sweetening agent, corrigent, smell masking agent, flavor, fluidizer (e.g., aerosil), antistatic agent, colorant, disintegrant, lubricant, plasticizer, anticoagulant, coating agent and the like can be mentioned. The additive is not particularly limited and, when the corresponding polymer mentioned above does not dissolve in the solvent to be used, the polymer does not exhibit the function in the present invention and is added as an additive.

For preparation of a medicament-containing particle, the average particle size of a polymer to be used as a starting material is not less than 5-fold, preferably not less than 10-fold, more preferably not less than 15-fold, further preferably not less than 20-fold, particularly preferably not less than 25-fold, that of the medicament used as a starting material and/or other additive. It is generally not more than 10000000-fold.

It is moreover preferable that the particle size distribution of a polymer used as a starting material should not overlap with the particle size distribution of a medicament used as a starting material and/or other additive. Specifically, for example, cumulative 10% particle size D10 of a polymer in a volume based measurement is preferably larger than cumulative 90% particle size D90 of the medicament and/or other additive. In other words, cumulative 10% particle size D10 of the polymer is preferably not less than 1-fold, more preferably not less than 2-fold, further preferably not less than 4-fold cumulative 90% particle size D90 of the medicament and/or other additive. It is generally not more than 5000000-fold.

When other additive is used, the average particle size of a mixed powder of a medicament used as a starting material and other additive is important for the preparation of a medicament-containing particle. In this case, the average particle size of a polymer used as a starting material is not less than 5-fold, preferably not less than 10-fold, more preferably not less than 15-fold, particularly preferably not less than 25-fold, that of a mixed powder of a medicament used as a starting material and other additive. It is generally not more than 1000-fold, preferably not more than 500-fold, more preferably not more than 100-fold.

It is moreover preferable that the particle size distribution of a polymer used as a starting material should not overlap with the particle size distribution of a mixed powder of a medicament used as a starting material and other additive. Specifically, for example, cumulative 10% particle size D10 of a polymer used as a starting material in a volume based measurement is preferably larger than cumulative 90% particle size D90 of a mixed powder of a medicament used as a starting material and other additive. In other words, cumulative 10% particle size D10 of the polymer used as a starting material is preferably not less than 1-fold, more preferably not less than 2-fold, further preferably not less than 4-fold cumulative 90% particle size D90 of a mixed powder of a medicament used as a starting material and other additive. It is generally not more than 500-fold, preferably not more than 250-fold, more preferably not more than 50-fold.

Medicament-containing particle of the present invention

The medicament-containing particle of the present invention is a particle composed of a shell (or wall) and a hollow, wherein the shell contains a medicament and a polymer. Alternatively, it is a particle having a structure wherein a hollow is surrounded by a wall composed of a composition comprising a medicament and a polymer.

The particle of the present inventions is characterized in that the particle has an inner hollow structure. The "hollow" here is different from the presence of many gaps at undetermined positions in general tablets, and refers to a completely independent single void present in the center of a particle, which is surrounded by the wall (shell) made of a medicament containing composition. For example, the presence thereof can be confirmed by an electron microscope or optical microscope.

The volume ratio of the hollow relative to the volume of the medicament-containing particle of the present invention as a whole is 1% - 50%, preferably 1% - 30%, more preferably 1.5% - 30%, particularly preferably about 2% - 30%. From another aspect, it is 4% - 50%, preferably 4% - 40%, more preferably 10% - 40%, further preferably about 10 - 30%. The volume ratio of a hollow is determined by dividing the volume of the hollow by the volume of the particle. Since the particle of the present inventions generally has high sphericity, the volume is determined by assuming that both the hollow and the particle are spheres. The volume of the hollow and the particle is calculated by determining the major diameter and the minor diameter of the hollow and the particle at the center of the particle by X ray CT (computerized tomography device), and determining the volume of the sphere assuming the average thereof to be hollow diameter and particle diameter.

To be specific, the "volume ratio of the hollow" in the present invention can be obtained by calculation by the following formula.

$$\text{volume ratio of hollow [\%]} = (4/3 \times \pi \times (\text{diameter of hollow}/2)^3)/(4/3 \times \pi \times (\text{particle size of medicament-containing particle}/2)^3) \times 100$$

The particle size of the medicament-containing particle and the diameter of the hollow are non-destructively measured by a benchtop micro-CT (manufactured by SKYSCAN, SKYSCAN1172) and the average of 10 measurements is used.

The medicament-containing particle of the present invention has a wall (shell) on the outside of the hollow. While the shell thickness can be freely determined, when the shell thickness is small, the strength of the particle becomes weak. The shell thickness of the present invention is preferably not less than 10 $\mu$m, more preferably not less than 15 $\mu$m, further preferably not less than 20 $\mu$m, most preferably not less than 30 $\mu$m. The shell thickness can be measured by, for example, X ray CT (computerized tomography device).

The percentage of the shell thickness may be any in the present invention, and can be determined by the following formula. It is preferably 20 - 80%, more preferably 30 - 70%.

$$\text{percentage of shell thickness [\%]} = (\text{shell thickness}/(\text{particle size of medicament-containing particle}/2)) \times 100$$

The medicament-containing particle of the present invention is characterized in that the particles size can be freely adjusted. Therefore, a particle having an average particle size of about 1 - 7000 $\mu$m, preferably about 5 - 1000 $\mu$m, more preferably about 10 - 500 $\mu$m, further preferably about 10 - 400 $\mu$m, still more preferably about 20 - 300 $\mu$m, particularly preferably about 50 - 300 $\mu$m, can be adjusted.

From the aspect of particle strength, a particle of preferably about 50 - 7000 $\mu$m, more preferably about 50 - 1000 $\mu$m, further preferably about 50 - 500 $\mu$m, from another aspect, a particle of preferably about 70 - 7000 $\mu$m, more preferably about 70 - 1000 $\mu$m, further preferably about 70 - 500 $\mu$m, particularly more preferably about 70 - 300 $\mu$m, most preferably about 100 - 300 $\mu$m, can be adjusted.

In the present invention, the size of the medicament-containing particle can be adjusted, as described above, by adjusting the average particle size of the polymer.

While the medicament-containing particle of the present invention has a hollow, the diameter of the hollow is generally not less than 10 $\mu$m. In addition, the diameter of the hollow can be freely adjusted to generally about 10 - 5000 $\mu$m, preferably about 20 - 700 $\mu$m, more preferably about 30 - 300 $\mu$m, further preferably about 50 - 200 $\mu$m. The hollow ratio can be freely changed, in association with the above-mentioned particle size.

In one embodiment, the medicament-containing particle of the present invention has a "smooth surface". As used herein, the smooth surface means absence of protrusion, and the surface does not have convex or concave. When medicament-containing particles are tableted, or filled in capsule and the like, the particles to be filled are required to have fluidity. Therefore, the medicament-containing particle preferably has a smooth surface. The medicament-containing particle also preferably has a smooth surface when coating is applied to impart functionality to the medicament-containing particle, since efficiency is improved. For example, such smoothness of the surface can be observed visually. For visual observation, a microscope and the like may be used for enlarged observation. The evaluation thereof is shown by "very smooth" (+++), "smooth" (++), "rather smooth" (+), and "not smooth" (-). "Very smooth" shows absence of a clear protrusion on the particle surface, and the surface does not have convex or concave. "Smooth" shows absence of a clear protrusion on the particle surface, but the surface has gentle concave or convex. "Rather smooth" shows presence of a clear protrusion or clear convex or concave on the particle surface. "Not smooth" shows presence of a clear protrusion and a clear convex or concave on the particle surface. The medicament-containing particle of the present invention may be "not smooth", preferably "very smooth", "smooth" or "rather smooth", more preferably "very smooth" or "smooth", further preferably "very smooth". 3D laser Scanning confocal microscope VK-X200 (KEYENCE) may be used for the measurement. The "smooth surface" specifically means that the surface roughness (Ra value) measured by the above-mentioned tool is not more than 3.5, preferably not more than 2.5, more preferably not more than 1.5.

The smoothness of the surface is influenced by the ratio of the average particle sizes of polymer and medicament and/or other additive. The average particle size of the polymer is not less than 5-fold, preferably not less than 10-fold, more preferably not less than 15-fold, further preferably not less than 20-fold, particularly preferably not less than 25-fold that of the average particle size of the medicament and/or other additive. It is generally not more than 1000-fold, preferably not more than 500-fold, more preferably not more than 100-fold.

In one embodiment, the medicament-containing particle of the present invention is spherical. As used herein, being "spherical" means having an aspect ratio of 1.0 - 1.5, preferably 1.0 - 1.4, more preferably 1.0 - 1.3. Having such shape, the medicament-containing particles show good fluidity when they are tableted, or filled in capsule and the like, and the efficiency is also improved during processing such as coating and the like.

The "aspect ratio" in the present invention is a ratio of the minor diameter and the major diameter of a particle, and is an indication of the sphericity. The aspect ratio can be determined by calculation by, for example, the following formula.

$$\text{aspect ratio} = \text{major diameter of particle/minor}$$

$$\text{diameter of particle}$$

The major diameter and minor diameter of the particle are non-destructively measured by a benchtop micro-CT (manufactured by SKYSCAN, SKYSCAN1172), and the average of 10 measurements is used.

In addition, Millitrac JPA (NIKKISO CO., LTD.) may be used for the measurement.

The "particle size distribution width" in the present invention can be obtained from the ratio of cumulative 90% particle size D90 and cumulative 10% particle size D10 (D90/D10) in the volume based measurement of a powder particle. The particle size distribution of the medicament-containing particle in the present invention can be conveniently adjusted by adjusting the particle size of the polymer and, for example, a particle group having a narrow particle size distribution width can be produced. Such particle size distribution width is measured by a laser diffraction particle size analyzer (manufactured by POWREX CORPORATION, Particle Viewer) by volume basis.

In the present invention, "width of particle size distribution is narrow" means that a specific particle size distribution width (D90/D10) is not more than 6.0, preferably not more than 5.0, more preferably not more than 4.0, further preferably not more than 3.0.

The strength of a hollow particle can be evaluated by a particle shell strength. The "particle shell strength" in the present invention can be obtained by calculation by the following formula.

particle shell strength [MPa]=$2.8P/(\pi \times d^2 - \pi \times d'^2) \times 1000$ P: destructive testing force of particles [mN], d: diameter of medicament-containing particle [$\mu$m], d' : diameter of hollow [$\mu$m]

The destructive testing force of the particle and the diameter of the medicament-containing particle are measured by SHIMADZU Corporation microcompression testing machine MCT-W500 (manufactured by Shimadzu Corporation).

The "diameter of hollow" in the present invention can be obtained by calculation by the following formula.

diameter of hollow [$\mu$m] = (major diameter of hollow + minor diameter of hollow)/2

The major diameter and minor diameter of the hollow of the particle are non-destructively measured by a benchtop micro-CT (manufactured by SKYSCAN, SKYSCAN1172) and the average of 10 measurements is used.

In the present invention, the medicament-containing particle is desired to have a sufficient particle strength, so that it will be efficiently coated without being broken or chipped, even when it is coated with a functional polymer and the like to impart an additional function by using a fluidized-bed granulator or various particulate coating machine and the like that require further mechanical strength of particles, and maintain the hollow without being crushed even after compression.

The particle of the present inventions has a sufficient particle strength. Since the particle has a hollow, a conventional particle strength measurement method cannot perform an accurate evaluation since it also calculates the hollow as a solid. Thus, the measurement is possible by the particle shell strength excluding the hollow. The "sufficient particle strength" in the present invention specifically means that the particle shell strength of the medicament-containing particle is not less than 2.0 MPa, preferably not less than 3.0 MPa, more preferably not less than 4.0 MPa, further preferably not less than 5.0 MPa.

The "particle size of medicament-containing particle" in the present invention can be obtained by calculation by the following formula.

The particle size of the medicament-containing particle can be obtained by calculation by the following formula.

particle size of medicament-containing particle [$\mu$m] = (major diameter of particle + minor diameter of particle)/2

The major diameter and minor diameter of the particle are non-destructively measured by a benchtop micro-CT (manufactured by SKYSCAN, SKYSCAN1172) and the average of 10 measurements is used.

The "shell thickness" in the present invention can be obtained by calculation by the following formula.

shell thickness [$\mu$m]=(particle size of medicament-containing particle-diameter of hollow)/2

The particle size of the medicament-containing particle and the diameter of the hollow are non-destructively measured by a benchtop micro-CT (manufactured by SKYSCAN, SKYSCAN1172) and the average of 10 measurements is used.

The "percentage of the shell thickness" in the present invention can be obtained by calculation by the following formula.

$$\text{percentage of shell thickness [\%]} = (\text{shell}$$

$$\text{thickness/(particle size of medicament-containing}$$

$$\text{particle/2))} \times 100$$

The particle size of the medicament-containing particle is non-destructively measured by a benchtop micro-CT (manufactured by SKYSCAN, SKYSCAN1172) and the average of 10 measurements is used.

The "volume ratio of hollow" in the present invention can be obtained by calculation by the following formula.

$$\text{volume ratio of hollow [\%]} = (4/3 \times \pi \times (\text{diameter of hollow}/2)^3)/(4/3 \times \pi \times (\text{particle size of medicament-containing particle}/2)^3) \times 100$$

The particle size of the medicament-containing particle and the diameter of the hollow are non-destructively measured by a benchtop micro-CT (manufactured by SKYSCAN, SKYSCAN1172) and the average of 10 measurements is used.

The "particle size distribution ratio (D50/D50) of polymer and medicament" in the present invention can be obtained by calculation by the following formula.

particle size distribution ratio of polymer and medicament (D50/D50) = D50 of polymer/D50 of medicament

The "particle size distribution ratio (D50/D50) of polymer and mixed powder of medicament and other additive" in the present invention can be obtained by calculation by the following formula

$$\text{particle size distribution ratio of polymer and mixed powder of medicament and other additive (D50/D50)} = \text{D50 of polymer/D50 of mixed powder of medicament and other additive}$$

The particle size distribution of polymer, medicament, and mixed powder of medicament and other additive is measured by a laser diffraction particle size analyzer (manufactured by POWREX CORPORATION, Particle Viewer) or a laser diffraction particle size analyzer (manufactured by Shimadzu Corporation, SALD-3000J) by volume basis.

The "particle size distribution ratio (D10/D90) of polymer and medicament" in the present invention can be obtained by calculation by the following formula.

particle size distribution ratio of polymer and medicament (D10/D90) = D10 of polymer/D90 of medicament

The "particle size distribution ratio (D10/D90) of polymer and mixed powder of medicament and other additive" in the present invention can be obtained by calculation by the following formula

$$\text{particle size distribution ratio of polymer and mixed powder of medicament and other additive (D10/D90)} = \text{D10 of polymer/D90 of mixed powder of medicament and other additive}$$

The particle size distribution of polymer, medicament, and mixed powder of medicament and other additive is measured by a laser diffraction particle size analyzer (manufactured by POWREX CORPORATION, Particle Viewer) or a laser diffraction particle size analyzer (manufactured by Shimadzu Corporation, SALD-3000J) by volume basis.

The medicament-containing particle of the present invention is useful as a medicament or a medicament starting material, and can be administered orally or parenterally to a human or animal. The dose can be appropriately selected according to the medicament to be used.

The medicament-containing particle of the present invention is generally used as a medicament or pharmaceutical composition containing a plurality of the medicament-containing particle.

The medicament-containing particle of the present invention can be formulated into various dosage forms according to the object of use. For example, the medicament-containing particle of the present invention can be used as it is, or granule, injection for preparation when in use, dosage form for implantation and the like. Moreover, it can be mixed with any additive and tableted to give a tablet (including orally disintegrating tablet), or filled in a capsule to give a capsule agent. Furthermore, the medicament-containing particle of the present invention can also be used as a suspension (aqueous suspension, oily suspension), emulsion and the like.

The present invention also relates to a process for preparation of a hollow particle comprising a step of granulating a powder mixture containing a medicament and a polymer while spraying a solvent capable of dissolving the polymer, and a hollow particle produced by the method.

Examples of the medicament, polymer, and the solvent capable of dissolving the polymer include those similar to the aforementioned examples recited for the process for preparation of the medicament-containing particle of the present invention. In the method, other additives may be contained as necessary, and Examples of other additive include those similar to the aforementioned examples recited for the process for preparation of the medicament-containing particle of the present invention.

Examples of the granulation method, drying method, solvent spray method and the like include those similar to the aforementioned examples recited for the process for preparation of the medicament-containing particle of the present invention.

Examples

The present invention is explained further specifically in the following by referring to Examples, Experimental Examples and Comparative Examples, which are not to be construed as limitative.

In the Examples, Experimental Examples and Comparative Examples, unless particularly indicated, % of solvent means (W/W%) and % of particle means wt%.

Unless particularly indicated, the additives used in the present Examples, Experimental Examples and Comparative Examples were the following.

hydroxypropylcellulose (HPC-L): Nippon Soda Co., Ltd.
hydroxypropylcellulose (HPC-SSL): Nippon Soda Co., Ltd.
hydroxypropylmethylcellulose (HPMC, TC5-R): Shin-Etsu Chemical Co., Ltd.
polyvinylpyrrolidone (PVP, plasdone K29-32): ISP Pharmaceuticals
polyvinyl alcohol (PVA, Gohsenol EG-05): The Nippon Synthetic Chemical Industry Co., Ltd.
pregelatinized starch (AMICOL C): NIPPON STARCH CHEMICAL CO., LTD.
aminoalkylmethacrylate copolymer RS (Eudragit RSPO): Evonik Degussa Japan Co., Ltd.
ethylcellulose (ETHOCEL 10P): The Dow Chemical Japan Company
Dried methacrylic acid copolymer LD (Eudragit L100-55): Evonik Degussa Japan Co., Ltd.
aminoalkylmethacrylate copolymer E (Eudragit E100): Evonik Degussa Japan Co., Ltd.
chitosan (FLONAC C-100M): Nippon Suisan Kaisha, Ltd.
D-mannitol (PEARLITOL 160C): ROQUETTE JAPAN
D-mannitol (PEARLITOL 200SD): ROQUETTE JAPAN
crystalline cellulose (CEOLUS KG-1000): Asahi Kasei Chemicals Corporation
crystalline cellulose (CEOLUS UF-711):Asahi Kasei Chemicals Corporation
cornstarch (cornstarch XX16): NIHON SHOKUHIN KAKO CO., LTD.
rice starch: Japan Corn Starch Co., Ltd.
magnesium carbonate (light): Kyowa Chemical Industry Co., Ltd.
low-substituted hydroxypropylcellulose (LH-21): Shin-Etsu Chemical Co., Ltd.
carmellose (NS-300): GOTOKU CHEMICAL CO., LTD.
croscarmellose sodium (Ac-Di-Sol SD-711): FMC Corporation
aspartame (aspartame): Ajinomoto Co., Inc.
neotame: DSP Gokyo Food & Chemical Co., Ltd.
aerosol (AEROSIL 200): NIPPON AEROSIL CO., LTD.
magnesium stearate (magnesium stearate): Taihei Chemical Industrial Co., Ltd.

The test methods in the present Examples, Experimental Examples and Comparative Examples are as described below.

(Particle size distribution)

The particle size distribution of medicament, polymer, other additive, a mixed powder of the medicament and other additive, and the obtained medicament-containing particle was measured by a laser diffraction particle size analyzer (manufactured by POWREX CORPORATION, Particle Viewer) or laser diffraction particle size analyzer (manufactured by Shimadzu Corporation, SALD-3000J) by volume basis.

(Appearance and cross-section of medicament-containing particle)

The appearance and cross-section of the particle were observed by a scanning electron microscope (manufactured by Hitachi, Ltd., S-3400N).

(Inner structure of medicament-containing particle)

The inner structure of the medicament-containing particle was non-destructively observed by a benchtop micro-CT (manufactured by SKYSCAN, SKYSCAN1172).

(Calculation of aspect ratio)

Unless particularly indicated, the aspect ratio of the obtained medicament-containing particle was obtained by non-destructively measuring the major diameter and minor diameter of the particle by a benchtop micro-CT (manufactured by SKYSCAN, SKYSCAN1172), and calculating by the following formula. The average of 10 measurements was used.

$$\text{Aspect ratio = major diameter of particle/minor diameter of particle}$$

(Measurement of particle strength of Comparative Examples)

The destructive testing force and particle size of the particles of Comparative Examples free of a hollow structure were measured by SHIMADZU Corporation micro-compression testing machine MCT-W500 (manufactured by Shimadzu Corporation), and the particle strength was calculated by the following formula (n=5).

$$\text{particle strength [MPa]} = 2.8P/(\pi \times d^2) \times 1000$$

P: destruction testing force of particle [mN], d: diameter of medicament-containing particle [$\mu$m]

(Measurement of particle shell strength)

The particle shell strength was determined by calculation by the following formula (n=5).

$$\text{particle shell strength [MPa]} = 2.8P/(\pi \times d^2 - \pi \times d'^2) \times 1000$$

P: destructive testing force of particle [mN], d: diameter of medicament-containing particle [$\mu$m], d': diameter of hollow [$\mu$m]
As the diameter of the hollow, a value calculated from the percentage of the shell thickness (measured and calculated using benchtop micro-CT description below) is used. That is, it is obtained by calculation by the following formula.

$$\text{diameter of hollow [μm] = diameter of medicament-containing particle} \times (1 - \text{percentage of shell thickness}/100)$$

The destructive testing force of the particle and the diameter of the medicament-containing particle are measured by SHIMADZU Corporation micro-compression testing machine MCT-W500 (manufactured by Shimadzu Corporation).

(Particle size of medicament-containing particle)

The particle size of the medicament-containing particle was determined by calculation by the following formula.

$$\text{particle size of medicament-containing particle [μm] =}$$
$$\text{(major diameter of particle + minor diameter of particle)}/2$$

The major diameter and minor diameter of the particle were non-destructively measured by a benchtop micro-CT (manufactured by SKYSCAN, SKYSCAN1172) and the average of 10 measurements was used.

(Diameter of hollow)

The diameter of hollow was determined by calculation by the following formula.

$$\texttt{diameter of hollow [µm] = (major diameter of hollow +}$$
$$\texttt{minor diameter of hollow)/2}$$

The major diameter and minor diameter of the hollow of the particle were non-destructively measured by a benchtop micro-CT (manufactured by SKYSCAN, SKYSCAN1172) and the average of 10 measurements was used.

(Shell thickness)

The shell thickness was determined by calculation by the following formula.

$$\texttt{shell thickness [µm] = (particle size of medicament-}$$
$$\texttt{containing particle - diameter of hollow)/2}$$

The particle size of the medicament-containing particle, and the diameter of the hollow were non-destructively measured by a benchtop micro-CT (manufactured by SKYSCAN, SKYSCAN1172) and the average of 10 measurements was used.

(Percentage of the shell thickness)

The "percentage of the shell thickness" in the present invention was determined by calculation by the following formula.

$$\texttt{percentage of shell thickness [\%]=(shell}$$
$$\texttt{thickness/(particle size of medicament-containing}$$
$$\texttt{particle/2))×100}$$

The particle size of the medicament-containing particle was non-destructively measured by a benchtop micro-CT (manufactured by SKYSCAN, SKYSCAN1172) and the average of 10 measurements was used.

(Volume ratio of hollow)

The volume ratio of the hollow was determined by calculation by the following formula.

$$\texttt{volume ratio of hollow [\%] = (4/3×π×(diameter of}$$
$$\texttt{hollow/2)}^3\texttt{)/(4/3×π×(particle size of medicament-containing}$$
$$\texttt{particle/2)}^3\texttt{)×100}$$

The particle size of the medicament-containing particle and the diameter of the hollow are non-destructively measured by a benchtop micro-CT (manufactured by SKYSCAN, SKYSCAN1172) and the average of 10 measurements was used.
(Particle size distribution ratio of polymer and medicament (D50/D50); particle size distribution ratio of polymer, and mixed powder of medicament and other additive (D50/D50))
The particle size distribution ratio of polymer and medicament (D50/D50) was determined by calculation by the following formula.

$$\text{particle size distribution ratio of polymer and}$$
$$\text{medicament (D50/D50)} = \text{D50 of polymer/D50 of medicament}$$

The particle size distribution ratio of polymer, and mixed powder of medicament and other additive (D50/D50) was determined by calculation by the following formula.

$$\text{particle size distribution ratio of polymer, and mixed}$$
$$\text{powder of medicament and other additive (D50/D50)} = \text{D50 of}$$
$$\text{polymer/D50 of mixed powder of medicament and other additive}$$

The particle size distribution of the polymer, medicament, and a mixed powder of the medicament and other additive was measured by a laser diffraction particle size analyzer (manufactured by POWREX CORPORATION, Particle Viewer) or laser diffraction particle size analyzer (manufactured by Shimadzu Corporation, SALD-3000J) by volume basis. (Particle size distribution ratio of polymer and medicament (D10/D90); particle size distribution ratio of polymer, and mixed powder of medicament and other additive (D10/D90))

The particle size distribution ratio of polymer and medicament (D10/D90) was determined by calculation by the following formula.

$$\text{particle size distribution ratio of polymer and}$$
$$\text{medicament (D10/D90)} = \text{D10 of polymer/D90 of medicament}$$

The particle size distribution ratio of polymer, and mixed powder of medicament and other additive (D10/D90) was determined by calculation by the following formula.

$$\text{particle size distribution ratio of polymer, and mixed}$$
$$\text{powder of medicament and other additive (D10/D90)} = \text{D10 of}$$
$$\text{polymer/D90 of mixed powder of medicament and other additive}$$

The particle size distribution of the polymer, medicament, and a mixed powder of the medicament and other additive was measured by a laser diffraction particle size analyzer (manufactured by POWREX CORPORATION, Particle Viewer) or laser diffraction particle size analyzer (manufactured by Shimadzu Corporation, SALD-3000J) by volume basis.

(Surface smoothness)

Observed by visual observation. The evaluation thereof is shown by "very smooth" (+++), "smooth" (++), "rather smooth" (+), and "not smooth" (-). "Very smooth" shows absence of a clear protrusion on the particle surface, and the surface does not have convex or concave. "Smooth" shows absence of a clear protrusion on the particle surface, but the surface has gentle concave or convex. "Rather smooth" shows presence of a clear protrusion or clear convex or concave on the particle surface. "Not smooth" shows presence of a clear protrusion and a clear convex or concave on the particle surface.

(Particle size distribution width)

The particle size distribution width was determined by calculation by the following formula.

$$\text{particle size distribution width} = \text{D90 of medicament-}$$
$$\text{containing particle/D10 of medicament-containing particle}$$

The particle size distribution of the medicament-containing particle was measured by a laser diffraction particle size analyzer (manufactured by POWREX CORPORATION, Particle Viewer) by volume basis.

Example 1

<Kind of medicament>

According to the formulation ratios and charge amounts in Table 1, medicament-containing particles of Examples 1-1 - 1-7 were produced. The medicaments used (all jet mill pulverized products) were zonisamide (1,2-benzisoxazole-3-methanesulfonamide, hereinafter Compound A), lurasidone hydrochloride ((3aR,4S,7R,7aS)-2-{(1R,2R)-2-[4-(1,2-benzisothiazol-3-yl)piperazin-1-ylmethyl]cyclohexylmethyl}hexahydro-4,7-methano-2H-isoindole-1,3-dione hydrochloride, hereinafter Compound B), metformin hydrochloride (1,1-dimethylbiguanide monohydrochloride, hereinafter, Compound C (Shin Nippon Yakugyo Co., Ltd.)), mesalazine (5-amino-2-hydroxybenzoic acid, hereinafter Compound D (Shin Nippon Yakugyo Co., Ltd.)), 3-[(1S)-1-(2-fluorobiphenyl-4-yl)ethyl]-5-{[amino(morpholin-4-yl)methylene]amino}isoxazole (hereinafter Compound E) and 5-(3-methoxyphenyl)-3-(5-methyl-1,2,4-oxadiazol-3-yl)-2-oxo-1,2-dihydro-1,6-naphthyridine (hereinafter Compound F).

As the polymer, particle size controlled product (100-165 mesh fraction or 165-200 mesh fraction) of hydroxypropylcellulose (HPC-L) in powdery condition was charged in the following granulator at 10 wt% relative to the total charge amount. Examples 1-1 - 1-6 were granulated for 30 min by a high shear granulator (vertical granulator, VG) FM-VG-05 (volume: 5 L, manufactured by POWREX CORPORATION) under the preparation conditions shown in Table 2 while spraying 50% ethanol or 20% ethanol aqueous solution (solvent), and fluidized-bed dried by multiplex MP-01 (manufactured by POWREX CORPORATION) to give particles containing 90 wt% of each medicament. Example 1-7 was granulated for 24 min by a high shear granulator (vertical granulator, VG) FM-VG-100 (volume: 100 L, manufactured by POWREX CORPORATION) under the preparation conditions shown in Table 3 while spraying purified water (solvent), and fluidized-bed dried by FLOW COATER FLO-5 (manufactured by Freund Corporation) to give a particle containing 90 wt% of the medicament.

The obtained particles were confirmed to be hollow, and the diameter of the hollow is shown in Table 37-1.

The particle size distribution of the medicament used was measured by a laser diffraction particle size analyzer (manufactured by Shimadzu Corporation, SALD-3000J), the particle size distribution of the polymer used was measured by a laser diffraction particle size analyzer (manufactured by POWREX CORPORATION, Particle Viewer), and the values are shown in Table 4.

Table 1

| | Example 1-1 | | Example 1-2 | | Example 1-3 | | Example 1-4 | | Example 1-5 | | Example 1-6 | | Example 1-7 | |
|---|---|---|---|---|---|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) | formulation ratio (%) | charge amount (g) | formulation ratio (%) | charge amount (g) | formulation ratio (%) | charge amount (g) |
| Compound A | 90 | 630 | - | - | - | - | - | - | - | - | - | - | - | - |
| Compound B | - | - | 90 | 630 | - | - | - | - | - | - | - | - | - | - |
| Compound C | - | - | - | - | 90 | 630 | - | - | - | - | - | - | - | - |
| Compound D | - | - | - | - | - | - | 90 | 630 | - | - | - | - | - | - |
| Compound E | - | - | - | - | - | - | - | - | 90 | 540 | - | - | - | - |
| Compound F | - | - | - | - | - | - | - | - | - | - | 90 | 630 | 90 | 12600 |
| hydroxypropylcellulose (100-165 mesh) | 10 | 70 | - | | 10 | 70 | 10 | 70 | 10 | 60 | 10 | 70 | 10 | 1400 |
| hydroxypropylcellulose (165-200 mesh) | - | - | 10 | 70 | - | - | - | - | - | - | - | - | - | - |
| (50% ethanol) | (23) | (160) | (36) | (252) | (23) | (164) | - | - | (30) | (180) | - | - | - | - |
| (20% ethanol) | - | - | - | - | - | - | (26) | (182) | - | - | (19) | (135) | - | - |
| (purified water) | - | - | - | - | - | - | - | - | - | - | - | - | (11) | (1600) |
| Total | 100 | 700 | 100 | 700 | 100 | 700 | 100 | 700 | 100 | 600 | 100 | 700 | 100 | 14000 |
| In the Table, the formulation ratio (%) is in wt%. | | | | | | | | | | | | | | |

Table 2

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| Pre-mixing | 3 | powder addition | - | 400 | 3,000 |
| granulation | 30 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

Table 3

| process | time (min) | polymer addition method | solvent addition method | blade rotation speed (rpm) | chopper rotation speed (rpm) |
|---|---|---|---|---|---|
| mixing, granulator | vertical granulator FM-VG-100 | | | | |
| Pre-mixing | 3 | powder addition | - | 150 | 3,000 |
| granulation | 24 | | spray (100 g/min) | 150 | 3,000 |
| dryer | FLOW COATER FLO-5 inlet air temperature; 70°C outlet air temperature; completed at 35°C | | | | |
| In the Table, (min) shows (minutes). | | | | | |

[Table 4]

| | particle size distribution ($\mu$m) | | |
|---|---|---|---|
| | D10 | D50 | D90 |
| Compound A | 0.7 | 2.7 | 5.6 |
| Compound B | 0.5 | 1.5 | 3.3 |
| Compound C | 1.0 | 3.2 | 5.2 |
| Compound D | 1.3 | 4.6 | 12.7 |
| Compound E | 1.3 | 3.2 | 6.3 |
| Compound F | 0.6 | 2.5 | 5.0 |
| hydroxypropylcellulose (100-165 mesh) | 81.2 | 137.8 | 264.3 |
| hydroxypropylcellulose (165-200 mesh) | 61.7 | 98.0 | 175.8 |

The appearance and cross-section of the produced particles were observed by a scanning electron microscope (S-3400N manufactured by Hitachi, Ltd.). The appearance of the particles is shown in Figs. 1-1 to 1-6, and the cross-section is shown in Figs. 1-7 to 1-9. As observed in Figs. 1-1 to 1-6, spherical particles having extremely high sphericity could be produced by using any medicaments. As observed in Figs. 1-7 to 1-9, the hollow structure was found in the cross-section. As a representative case, the minor and major diameters of the particles obtained in Example 1-1 and 1-4 were measured by Millitrac JPA (manufactured by NIKKISO CO., LTD.) (n=1), and the aspect ratios were calculated by the formula: aspect ratio = major diameter/minor diameter. The respective values were 1.22 and 1.25, which clarified that the particles were spherical.

Then, the particle size distribution of the produced spherical particles was measured by Particle Viewer (manufactured by POWREX CORPORATION). The results are shown in Table 5. As shown in Table 5, even when any medicaments were used, spherical particles having extremely narrow particle size distribution width (ratio of cumulative 90% particle size D90 and cumulative 10% particle size D10 (D90/D10) in volume based measurement of powder particles) could be produced without limitation on the medicaments.

[Table 5]

| | | particle size distribution (μm) | | | particle size distribution width |
|---|---|---|---|---|---|
| | | D10 | D50 | D90 | D90/D10 |
| Example 1-1 | spherical particles containing 90% of Compound A | 115 | 175 | 239 | 2.1 |
| Example 1-2 | spherical particles containing 90% of Compound B | 95 | 149 | 215 | 2.3 |
| Example 1-3 | spherical particles containing 90% of Compound C | 111 | 168 | 233 | 2.1 |
| Example 1-4 | spherical particles containing 90% of Compound D | 114 | 182 | 261 | 2.3 |
| Example 1-5 | spherical particles containing 90% of Compound E | 117 | 179 | 247 | 2.1 |
| Example 1-6 | spherical particles containing 90% of Compound F | 106 | 169 | 238 | 2.2 |
| Example 1-7 | spherical particles containing 90% of Compound F | 114 | 199 | 295 | 2.6 |

The relationship between the particle size distribution of the polymer (hydroxypropylcellulose) and the particle size distribution of the medicament-containing particle in Example 1-6 is shown in Fig. 1-10. As shown in Fig. 1-10, the particle size distribution of the medicament-containing particle was confirmed to reflect the particle size distribution of the polymer.

Example 2

<Amount of hydroxypropylcellulose added>

According to the formulation ratio and charge amount of Table 6, a jet mill pulverized product of Compound A as a medicament and a particle size controlled product of hydroxypropylcellulose (HPC-L) (100-165 mesh fraction) as a polymer in powder were charged in a high shear granulator (vertical granulator, VG) (FM-VG-05, volume: 5 L, manufactured by POWREX CORPORATION) at 5, 15 and 30 wt% relative to the total charge amount. Under the preparation conditions shown in Table 7, they were granulated for 20 - 30 min while spraying purified water or 50% ethanol aqueous solution (solvent), and fluidized-bed dried by using multiplex MP-01 (manufactured by POWREX CORPORATION) to give Compound A-containing spherical particles of Examples 2-1, 2-2 and 2-3. The obtained particles were confirmed to be hollow, and the diameter of the hollow is shown in Table 37-1.

The particle size distribution of the medicament was measured by a laser diffraction particle size analyzer (manufactured by Shimadzu Corporation, SALD-3000J), and the particle size distribution of the polymers used was measured by a laser diffraction particle size analyzer (manufactured by POWREX CORPORATION, Particle Viewer). The values are shown in Table 4. In addition, the formulation of Example 1-1 and the particle size distribution of the medicament-containing particles are described in Tables 6 and 8.

[Table 6]

| | Example 2-1 | | Example 1-1 | | Example 2-2 | | Example 2-3 | |
|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) |
| Compound A | 95 | 665 | 90 | 630 | 85 | 595 | 70 | 420 |
| hydroxypropylcellulose | 5 | 35 | 10 | 70 | 15 | 105 | 30 | 180 |
| (purified water) | (10) | (70) | - | - | (16) | (112) | (18) | (105) |
| (50% ethanol) | - | - | (23) | (160) | - | - | - | - |
| Total | 100 | 700 | 100 | 700 | 100 | 700 | 100 | 600 |
| In the Table, the formulation ratio (%) is in wt%. | | | | | | | | |

[Table 7]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 20-30 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

The particle size distribution of the produced particles was measured by Particle Viewer (manufactured by POWREX CORPORATION). The results are shown in Table 8. As shown in Table 8, when the amount of the polymer was 5 - 30 wt%, particles having extremely narrow particle size distribution width (ratio of cumulative 90% particle size D90 and cumulative 10% particle size D10 (D90/D10) in volume based measurement of powder particles) could be produced.

[Table 8]

| | proportion of hydroxypropylcellulose (%) | particle size distribution ($\mu$m) | | | particle size distribution width |
|---|---|---|---|---|---|
| | | D10 | D50 | D90 | D90/D10 |
| Example 2-1 | 5 | 47 | 170 | 259 | 5.5 |
| Example 1-1 | 10 | 115 | 175 | 239 | 2.1 |
| Example 2-2 | 15 | 110 | 167 | 232 | 2.1 |
| Example 2-3 | 30 | 91 | 151 | 221 | 2.4 |
| In the Table, (%) shows wt%. | | | | | |

The appearance and cross-section of the produced particles were observed under a scanning electron microscope (S-3400N manufactured by Hitachi, Ltd.). The appearance of the spherical particles is shown in Figs. 2-1 and 2-2, and the cross-section is shown in Figs. 2-3 to 2-5. As observed in Figs. 2-1 and 2-2, spherical particles having extremely high sphericity could be produced. As observed in Figs. 2-3 to 2-5, the hollow structure was found in the cross-section.

Example 3

<Particles using various polymers>

According to the formulation ratios and charge amounts in Tables 9-1 and 9-2, a jet mill pulverized product of Compound A as a medicament and, as polymers, hydroxypropylcellulose (HPC-L, 100-165 mesh fraction), hydroxypropylmethylcellulose (200 mesh on product), polyvinylpyrrolidone (200 mesh on product), polyvinyl alcohol (60-140 mesh fraction) and pregelatinized starch (100-200 mesh fraction), which are water-soluble polymers, and aminoalkylmethacrylate copolymer RS (100-140 mesh fraction), ethylcellulose (80 mesh pass product), which are water insoluble polymers, and dried methacrylic acid copolymer LD (200 mesh on product), which is an enteric polymer, each in powder, were charged in a high shear granulator (vertical granulator, VG) FM-VG-05 (volume: 5 L, manufactured by POWREX CORPORATION). Under the preparation conditions shown in Table 10, they were granulated for 20 - 45 min while spraying purified water, 50% ethanol aqueous solution, 80% ethanol aqueous solution or 95% ethanol aqueous solution (solvent), and fluidized-bed dried by multiplex MP-01 (manufactured by POWREX CORPORATION) to give Compound A-containing spherical particles of Examples 3-1 - 3-7. The obtained particles were confirmed to be hollow, and the diameter of the hollow is shown in Table 37-1.

The particle size distribution of the medicament used was measured by a laser diffraction particle size analyzer (manufactured by Shimadzu Corporation, SALD-3000J), and the particle size distribution of the polymers used was measured by a laser diffraction particle size analyzer (manufactured by POWREX CORPORATION, Particle Viewer), and the values are shown in Table 11-1. The formulation of Example 1-1 is described in Table 9-1, and the particle size distribution of the medicaments, the polymers and the medicament-containing particles are described in Tables 11-1 and 11-2.

[Table 9-1]

| | Example 1-1 | | Example 3-1 | | Example 3-2 | | Example 3-3 | | Example 3-4 | |
|---|---|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) ( | formulation ratio (%) | charge amount (g) |
| Compound A | 90 | 630 | 90 | 540 | 90 | 630 | 90 | 540 | 80 | 480 |
| hydroxypropylcellulose | 10 | 70 | - | - | - | - | - | - | - | - |
| hydroxypropylmethylcellulose | - | - | 10 | 60 | - | - | - | - | - | - |
| polyvinylpyrrolidone | - | - | - | - | 10 | 70 | - | - | - | - |
| polyvinyl alcohol | - | - | - | - | - | - | 10 | 60 | - | - |
| pregelatinized starch | - | - | - | - | - | - | - | - | 20 | 120 |
| (purified water) | - | - | - | - | (11) | (75) | (30) | (180) | (53) | (315) |
| (50% ethanol) | (23) | (160) | - | - | - | - | - | - | - | - |
| (80% ethanol) | - | - | (38) | (225) | - | - | - | - | - | - |
| Total | 100 | 700 | 100 | 600 | 100 | 700 | 100 | 600 | 100 | 600 |
| In the Table, the formulation ratio (%) is in wt%. | | | | | | | | | | |

EP 3 791 867 A1

28

[Table 9-2]

| | Example 3-5 | | Example 3-6 | | Example 3-7 | |
|---|---|---|---|---|---|---|
| | formulation ratio (%) | charge amount (g) | formulation ratio (%) | charge amount (g) | formulation ratio (%) | charge amount (g) |
| Compound A | 80 | 560 | 90 | 630 | 80 | 560 |
| aminoalkylmethacrylate copolymer RS | 20 | 140 | - | - | - | - |
| ethylcellulose | - | - | 10 | 70 | - | - |
| dried methacrylic acid copolymer LD | - | - | - | - | 20 | 140 |
| (95% ethanol) | (30) | (210) | (34) | (240) | (34) | (235) |
| Total | 100 | 700 | 100 | 700 | 100 | 700 |
| In the Table, the formulation ratio (%) is in wt%. | | | | | | |

[Table 10]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 20-45 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 11-1]

| | particle size distribution (μm) | | |
|---|---|---|---|
| | D10 | D50 | D90 |
| Compound A | 0.7 | 2.7 | 5.6 |
| hydroxypropylcellulose (100-165 mesh) | 81.2 | 137.8 | 264.3 |
| hydroxypropylmethylcellulose (200 mesh on) | 73.1 | 131.3 | 253.5 |
| polyvinylpyrrolidone (200 mesh on) | 74.1 | 138.4 | 303.9 |
| polyvinyl alcohol (60-140 mesh) | 117.1 | 190.6 | 282.6 |
| pregelatinized starch (100-200 mesh) | 99.3 | 144.2 | 238.1 |
| aminoalkylmethacrylate copolymer RS (100-140 mesh) | 112.0 | 145.1 | 190.4 |
| ethylcellulose (80 mesh pass) | 112.6 | 171.0 | 260.7 |
| dried methacrylic acid copolymer LD (200 mesh on) | 32.8 | 70.1 | 212.6 |

As a representative case, the inner structure of the medicament-containing particle produced in Example 3-5 was non-destructively observed by a benchtop micro-CT (manufactured by SKYSCAN, SKYSCAN1172) (Fig. 3). As shown in Fig. 3, it was confirmed that hollow medicament-containing particles could be prepared.

The particle size distribution of the particles produced was measured by Particle Viewer (manufactured by POWREX CORPORATION). The results are shown in Table 11-2.

[Table 11-2]

| Example | particle size distribution (μm) | | | particle size distribution width D90/D10 |
|---|---|---|---|---|
| | D10 | D50 | D90 | |
| 1-1 | 115 | 175 | 239 | 2.1 |
| 3-1 | 95 | 157 | 230 | 2.4 |
| 3-2 | 117 | 283 | 640 | 5.5 |
| 3-3 | 108 | 231 | 552 | 5.1 |
| 3-4 | 95 | 169 | 265 | 2.8 |
| 3-5 | 110 | 191 | 276 | 2.5 |
| 3-6 | 58 | 128 | 256 | 4.4 |
| 3-7 | 68 | 122 | 186 | 2.7 |

Example 4

<Other additives>

According to the formulation ratios and charge amounts in Table 12, Compound A and Compound B (both jet mill pulverized products) as a medicament, and hydroxypropylcellulose (HPC-L) (Example 4-1: without particle size control, Example 4-2: 200-325 mesh fraction) as a polymer, and other additives shown in Table 12 in powder were charged in a high shear granulator (vertical granulator, VG) FM-VG-05 (volume: 5 L, manufactured by POWREX CORPORATION). Under the preparation conditions shown in Table 13, they were granulated for 23-31 min while spraying 50% ethanol aqueous solution or 80% ethanol aqueous solution, and fluidized-bed dried by multiplex MP-01 (manufactured by POWREX CORPORATION) to give spherical medicament containing particle of Examples 4-1 and 4-2. The obtained particles were confirmed to be hollow, and the diameter of the hollow is shown in Table 37-1.
The particle size distribution of the medicaments used, other additives, and mixed powders of the medicament and other additives was measured by a laser diffraction particle size analyzer (manufactured by Shimadzu Corporation, SALD-3000J), and the particle size distribution of the polymers used was measured by a laser diffraction particle size analyzer (manufactured by POWREX CORPORATION, Particle Viewer). The values are shown in Table 14-1.

[Table 12]

| | Example 4-1 | | Example 4-2 | |
|---|---|---|---|---|
| | formulation ratio (%) | charge amount (g) | formulation ratio (%) | charge amount (g) |
| Compound A | 80 | 560 | - | - |
| Compound B | - | - | 80 | 560 |
| magnesium carbonate (light) | 10 | 70 | - | - |
| rice starch | - | - | 10 | 70 |
| hydroxypropylcellulose | 10 | 70 | 9.5 | 66.5 |
| aerosil | - | - | 0.5 | 3.5 |
| (80% ethanol) | (22) | (155) | - | - |
| (50% ethanol) | - | - | (29) | (205) |
| Total | 100 | 700 | 100 | 700 |
| In the Table, the formulation ratio (%) is in wt%. | | | | |

[Table 13]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 23-31 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 14-1]

| Example 4-1 | particle size distribution (pm) | | |
|---|---|---|---|
| | D10 | D50 | D90 |
| Compound A | 0.7 | 2.7 | 5.6 |
| magnesium carbonate (light) | 0.9 | 4.0 | 10.6 |
| hydroxypropylcellulose (not fractionated) | 70.1 | 179.5 | 349.8 |
| mixed powder of Compound A and magnesium carbonate | 1.2 | 3.0 | 5.4 |
| Example 4-2 | particle size distribution ($\mu$m) | | |
| | D10 | D50 | D90 |
| Compound B | 0.5 | 1.5 | 3.3 |
| Hydroxypropylcellulose (200-325 mesh) | 39.3 | 69.8 | 108.9 |
| rice starch | 4.3 | 13.2 | 64.1 |
| aerosil | N.P. | N.P. | N.P. |
| mixed powder of Compound B, rice starch and aerosil | 0.6 | 2.2 | 5.6 |
| N.P.: Not Performed | | | |

The particle size distribution of the produced particles was measured by Particle Viewer (manufactured by POWREX CORPORATION). The results are shown in Table 14-2.

[Table 14-2]

| Example | particle size distribution ($\mu$m) | | | particle size distribution width D90/D10 |
|---|---|---|---|---|
| | D10 | D50 | D90 | |
| 4-1 | 114 | 228 | 434 | 3.8 |
| 4-2 | N.P. | N. P. | N.P. | N.P. |
| N.P.: Not Performed | | | | |

Example 5 Tablet

Using the medicament-containing particles produced in Examples 3-5 and 4-2 and according to the formulation amounts and charge amounts in Table 15, additives were mixed and the mixture was tableted to give tablets of Examples 5-1 and 5-2.

To be specific, in Example 5-1, according to the charge amount in Table 15, the medicament-containing particle and the additive were weighed, and mixed by a micro v-shaped mixer (Tsutsui Scientific Instruments Co., Ltd.) at 30 rpm (rotation speed) for 5 min. The mixed product was tableted by a rotary tableting machine (VEL2: Kikusui Seisakusho Ltd.) at 20 rpm (rotation speed) (flat tablet with beveled edge, $\varphi$8.0 mm, tableting pressure: 11-12 kN). The produced tablet was subjected to a dissolution test according to Experimental Example 1.

In Example 5-2, according to the charge amount in Table 15, the medicament-containing particle and additive were weighed, mixed in a plastic bag, and tableted by a simple molding machine (table press TB-20H, NPa system) (flat tablet, $\varphi$7.5 mm, tableting pressure: 8 kN). The produced tablet was subjected to a dissolution test according to Experimental Example 2.

Furthermore, the mixed powder before tableting, which was produced in the above-mentioned Example 5-1, was tableted by a simple molder (table press TB-20H, NPa system) (flat tablet with beveled edge, $\varphi$8.0 mm, tableting pressure: 5kN) to give the tablet of Example 5-3. The produced tablet was divided, and the cross-section thereof was observed under a scanning electron microscope (manufactured by Hitachi, Ltd., S-3400N). As a result, the presence of a hollow particle shown in Fig. 4 could be confirmed.

[Table 15]

| | Example 5-1 | | Example 5-2 | |
|---|---|---|---|---|
| | formulation amount (mg) | charge amount (g) | formulation amount (mg) | charge amount (g) |
| Compound A-containing particles (Example 3-5) | 31.8 | 144.9 | - | - |
| Compound B-containing particles (Example 4-2) | - | - | 100.0 | 50.0 |
| D-mannitol (PEARLITOL 160C) | 95.2 | 434.2 | - | - |
| D-mannitol (PEARLITOL 200SD) | - | - | 34.4 | 17.2 |
| crystalline cellulose (CEOLUS KG-1000) | 40.0 | 182.4 | - | - |
| crystalline cellulose (CEOLUS UF-711) | - | - | 16.0 | 8.0 |
| cornstarch | 20.0 | 91.2 | - | - |
| low-substituted hydroxypropylcellulose | 6.0 | 27.4 | - | - |
| carmellose | 4.0 | 18.2 | - | - |
| croscarmellose sodium | - | - | 8.0 | 4.0 |
| aspartame | 1.0 | 4.6 | - | - |
| magnesium stearate | 2.0 | 9.1 | 1.6 | 0.8 |
| Total | 200 | 912 | 160 | 80 |

(Experimental Example 1)

A dissolution test was performed using the tablet of Example 5-1. According to Dissolution Test Method Paddle Method based on the Japanese Pharmacopoeia, 15th Edition, the measurement was performed at 50 rpm (rotation speed) using purified water (37°C/900 mL) or 2nd fluid for dissolution test (about pH 7) as a test solution. The measurement time was 5, 10, 15, 30, 45 and 60 min. Sampling liquid was passed through a filter and measured by an ultraviolet visible absorption spectrophotometer, based on which the dissolution rate was calculated.

<Measurement wavelength>

measurement wavelength: 285 nm

(Experimental Example 2)

A dissolution test was performed using the tablet of Example 5-2. Based on Dissolution Test Method Paddle Method of the Japanese Pharmacopoeia, 15th Edition, and using McIlvaine buffer (pH 3.8, 37°C/900 mL) as a test solution, the measurement was performed at 50 rpm (rotation speed). The measurement time was 5, 15, 30, 45 or 60 min, and the sampling liquid was passed through a filter and measured by HPLC, based on which the dissolution rate was calculated.

<HPLC measurement conditions>

detector: ultraviolet absorption spectrophotometer
measurement wavelength: 230 nm
column: Onyx Monolithic C18 (4.6 mmΦ×100 mmL)
column temperature: 40°C
flow rate: 2.5 mL/mim
injection volume: 25 μL
sample cooler: 25°C
syringe washing: water/acetonitrile mixed solution=1/1
mobile phase: diluted phosphoric acid (1→1000)/acetonitrile mixed solution (3:2)

The results of the dissolution test of the tablets obtained in Examples 5-1 and 5-2 are shown in Table 16. The tablet produced using aminoalkylmethacrylate copolymer RS, which is a water-insoluble polymer, showed moderate dissolution in both the 2nd fluid for dissolution test and purified water (Example 5-1). The tablet produced using hydroxypropylcellulose, which is a water-soluble polymer, showed rapid dissolution (Example 5-2). From these Examples, polymer functionality was confirmed to have been imparted.

[Table 16]

| Dissolution rate (%) | | | |
|---|---|---|---|
| time (min) | Example 5-1 | | Example 5-2 |
| | 2nd fluid for dissolution test | purified water | McIlvaine buffer pH 3.8 |
| 0 | 0 | 0 | 0 |
| 5 | 29 | 29 | 63 |
| 10 | 49 | 48 | - |
| 15 | 63 | 62 | 88 |
| 30 | 89 | 87 | 98 |
| 45 | 100 | 101 | 101 |
| 60 | 103 | 105 | 102 |
| In the Table, (min) shows (minutes). | | | |

Comparative Example 1

Spherical particles were obtained by the method disclosed in patent document 1. That is, acetaminophen (80 parts) pulverized by a hammer mill (manufactured by POWREX CORPORATION), and crystalline cellulose (19 parts, trade name: CEOLUS PH-F20JP manufactured by Asahi Kasei Corporation) were charged in a high shear granulator (vertical granulator, VG) (FM-VG-05, volume: 5 L, manufactured by POWREX CORPORATION), and mixed well. The mixture was granulated for 25 min by agitating at 25°C, 400 rpm while adding a solution of hydroxypropylcellulose (trade name: HPC-SL, manufactured by Nippon Soda Co., Ltd., 1 part) dissolved in a mixed solution of ethanol (79 parts) and water (20 parts). After the granulation, the granules were dried by shelf dryer at 45°C for 3 hr to give spherical particles. The inner structure of the produced spherical particles was non-destructively observed by a benchtop micro-CT (SKYSCAN1172 manufactured by SKYSCAN). The inner structure of the particles is shown in Fig. 5, in which a hollow was not found. The particle strength was 2.3 (MPa).
The particle size distribution of the medicament used (acetaminophen) was measured by a laser diffraction particle size analyzer (manufactured by Shimadzu Corporation, SALD-3000J), and the particle size distribution of other additive used (crystalline cellulose (CEOLUS PH-F20JP)) was measured by a laser diffraction particle size analyzer (manufactured by POWREX CORPORATION, Particle Viewer). The values are shown in Table 17.

[Table 17]

|  | particle size distribution (μm) | | |
| --- | --- | --- | --- |
|  | D10 | D50 | D90 |
| acetaminophen | 2.8 | 11.6 | 46.1 |
| crystalline cellulose (CEOLUS PH-F20JP) | 6.0 | 18.4 | 44.3 |

Example 6 and Comparative Example 2

<Effect of polymer (1)>

According to the formulation ratio and charge amount of Table 18-1, medicament-containing particles of Examples 6-1 to 6-4 were produced. A jet mill pulverized product of Compound A as a medicament and, as polymers, dried methacrylic acid copolymer LD (100-150 mesh fraction) which is an enteric polymer, aminoalkylmethacrylate copolymer E (pulverized by Fitz Mill (screen size: 42 mesh) and 60 - 100 mesh fraction was used) which is a gastric soluble polymer, aminoalkylmethacrylate copolymer RS (100 mesh on product) which is a sustained-release polymer, and hydroxypropylcellulose (HPC-L) (100-150 mesh fraction) which is a water-soluble polymer were weighed, and they were charged in a high shear granulator (vertical granulator, VG) (FM-VG-05, volume: 5 L, manufactured by POWREX CORPORATION) each in a powdery condition. Under the preparation conditions shown in Table 19-1, they were granulated for 32 - 47 min while spraying 95% ethanol aqueous solution (solvent), and fluidized-bed dried by using multiplex MP-01 (manufactured by POWREX CORPORATION) to give Compound A-containing particles. The obtained particles were confirmed to be hollow, and the diameter of the hollow is shown in Table 37-2.

According to the formulation ratios and charge amounts in Table 18-2, medicament-containing particles of Comparative Examples 2-1 to 2-5 were produced. A jet mill pulverized product of Compound A as a medicament and, as polymers, dried methacrylic acid copolymer LD (non-fractionated product) which is an enteric polymer, aminoalkylmethacrylate copolymer E (Eudragit EPO, non-fractionated product) which is a gastric soluble polymer, aminoalkylmethacrylate copolymer RS (non-fractionated product) which is a sustained-release polymer, and hydroxypropylcellulose (HPC-L) (non-fractionated product) which is a water-soluble polymer were weighed and sufficiently mixed in a plastic bag. The mixed powders were dry-granulated by roller compactor TF-MINI (manufactured by Freund Corporation) under the preparation conditions shown in Table 19-2 to give Compound A-containing particles.

The particle size distribution of the polymers used was measured by a laser diffraction particle size analyzer (manufactured by POWREX CORPORATION, Particle Viewer), and the particle size distribution of the medicament used was measured by a laser diffraction particle size analyzer (manufactured by Shimadzu Corporation, SALD-3000J). The values are shown in Table 20-1. The formulation of Example 3-7 is described in Table 18-1, and the particle size distribution of the medicament, polymers and medicament-containing particles is described in Tables 20-1 and 20-2.

[Table 18-1]

| | Example 3-7 | | Example 6-1 | | Example 6-2 | | Example 6-3 | | Example 6-4 | |
|---|---|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) | formulation ratio (%) | charge amount (g) |
| Compound A | 80 | 560 | 80 | 560 | 80 | 560 | 80 | 560 | 80 | 560 |
| dried methacrylic acid copolymer LD (200 mesh on) | 20 | 140 | | | | | | | | |
| dried methacrylic acid copolymer LD (100-150 mesh) | | | 20 | 140 | | | | | | |
| aminoalkylmethacrylate copolymer E (60-100 mesh) | | | | | 20 | 140 | | | | |
| aminoalkylmethacrylate copolymer RS (100 mesh on) | | | | | | | 20 | 140 | | |
| hydroxypropylcellulose (100-150 mesh) | | | | | | | | | 20 | 140 |
| (95% ethanol) | (34) | (235) | (54) | (380) | (42) | (292) | (46) | (325) | (30) | (210) |
| Total | 100 | 700 | 100 | 700 | 100 | 700 | 100 | 700 | 100 | 700 |
| In the Table, the formulation ratio (%) is in wt%. | | | | | | | | | | |

[Table 18-2]

| | Example 3-7 | | Example 6-1 | | Example 6-2 | | Example 6-3 | | Example 6-4 | |
|---|---|---|---|---|---|---|---|---|---|---|
| | formulation ratio (%) | Charge amount (g) | formulation ratio (%) | charge amount (g) | formulation ratio (%) | charge amount (g) | formulation ratio (%) | charge amount (g) | formulation ratio (%) | charge amount (g) |
| Compound A | 80 | 560 | 80 | 560 | 80 | 560 | 80 | 560 | 80 | 560 |
| dried methacrylic acid copolymer LD (200 mesh on) | 20 | 140 | | | | | | | | |
| dried methacrylic acid copolymer LD (100-150 mesh) | | | 20 | 140 | | | | | | |
| aminoalkylmethacrylate copolymer E (60-100 mesh) | | | | | 20 | 140 | | | | |
| aminoalkylmethacrylate copolymer RS (100 mesh on) | | | | | | | 20 | 140 | | |
| hydroxypropylcellulose (100-150 mesh) | | | | | | | | | 20 | 140 |
| (95% ethanol) | (34) | (235) | (54) | (380) | (42) | (292) | (46) | (325) | (30) | (210) |
| Total | 100 | 700 | 100 | 700 | 100 | 700 | 100 | 700 | 100 | 700 |
| In the Table, the formulation ratio (%) is in wt%. | | | | | | | | | | |

[Table 19-1]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 32-57 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 19-2]

| process | feeder rotation speed (rpm) | roll rotation speed (rpm) | roll pressure (kgf/cm2) |
|---|---|---|---|
| granulation | 10 | 2-4 | 80-100 |

[Table 20-1]

| Example 6 | particle size distribution (μm) | | |
|---|---|---|---|
| | D10 | D50 | D90 |
| Compound A | 0.7 | 2.7 | 5.6 |
| dried methacrylic acid copolymer LD (200 mesh on) | 32.8 | 70.1 | 212.6 |
| dried methacrylic acid copolymer LD (100-150 mesh) | 55.1 | 101.2 | 152.1 |
| aminoalkylmethacrylate copolymer E (60-100 mesh) | 94.2 | 158.9 | 228.2 |
| aminoalkylmethacrylate copolymer RS (100 mesh on) | 127.2 | 194.6 | 281.3 |
| hydroxypropylcellulose (HPC-L) (100-150 mesh) | 88.2 | 138.4 | 202.6 |
| Comparative Example 2 | particle size distribution (μm) | | |
| | D10 | D50 | D90 |
| Compound A | 0.7 | 2.7 | 5.6 |
| dried methacrylic acid copolymer LD (not fractionated) | 22.4 | 47.0 | 79.1 |
| aminoalkylmethacrylate copolymer E (not fractionated) | 7.1 | 12.7 | 21.1 |
| aminoalkylmethacrylate copolymer RS (not fractionated) | 35.0 | 108.1 | 202.0 |
| hydroxypropylcellulose (HPC-L) (not fractionated) | 46.5 | 128.6 | 214.1 |

[Table 20-2]

| Example | particle size distribution (μm) | | | particle size distribution width D90/D10 |
|---|---|---|---|---|
| | D10 | D50 | D90 | |
| 3-7 | 68 | 122 | 186 | 2.7 |
| 6-1 | 133 | 196 | 272 | 2.0 |
| 6-2 | 150 | 229 | 323 | 2.2 |
| 6-3 | 218 | 306 | 420 | 1.9 |

(continued)

| Example | particle size distribution (μm) | | | particle size distribution width D90/D10 |
|---------|------|------|------|------|
| | D10 | D50 | D90 | |
| 6-4 | 135 | 193 | 255 | 1.9 |

(Experimental Example 3)

Using 30-140 mesh fractions of the medicament-containing particles produced in Examples 3-7 and 6-1 to 6-4, and Comparative Examples 2-1 to 2-5, a dissolution test was performed by the following steps. As a test solution, dissolution test 1st fluid (about pH 1.2) or 2nd fluid for dissolution test (about pH 7) of the Japanese Pharmacopoeia, 15th Edition, was used.

<Preparation of standard solution>

Reference standard of Compound A was dried at 105°C for 3 hr, and about 22 mg thereof was precisely weighed and dissolved in the test solution to accurately 200 mL. This solution (4 mL) was accurately weighed and the test solution was added to accurately 20 mL to give a standard solution.

<Preparation of sample solution>

Medicament-containing particles in an amount corresponding to 25 mg of Compound A was precisely weighed and used as a sample. Using a test solution (900 mL), the test was performed at 50 rpm according to Dissolution Test Method 2 of the Japanese Pharmacopoeia, 15th Edition. At 5, 10, 15, 30, 45, 60, 120, 180, 240 and 360 min from the start of the dissolution test, the eluate (20 mL) was sampled, and the same volume of the test solution heated to $37 \pm 0.5$°C was immediately supplemented with care. The eluate was filtered through a membrane filter (Millex-HA (registered trade mark)) having a pore size of 0.45 μm or less. The initial filtrate (about 10 mL) was removed, and the next filtrate was used as a sample solution.

<Analysis method>

The sample solution and standard solution were subjected to a test by an ultraviolet visible absorbance measurement method, the absorbance at wavelength 285 nm was measured, and the dissolution rate was calculated.

The results obtained using the 2nd fluid for dissolution test are shown in Figs. 6-1 to 6-4.

From Fig. 6-1, the dissolution of the medicament-containing particle (Comparative Example 2-2), which was dry-granulated using a functional polymer (dried methacrylic acid copolymer LD which is an enteric polymer), in 2nd fluid for dissolution test was almost equivalent to that of the medicament-containing particle (Comparative Example 2-1) free of a functional polymer, and the effect of the functional polymer was not exhibited. In contrast, the medicament-containing particle (Example 3-7) of the present invention showed very rapid dissolution, and exhibited the effect of the functional polymer.

From Fig. 6-2, the dissolution of the medicament-containing particle (Comparative Example 2-3), which was dry-granulated using a functional polymer (dried methacrylic acid copolymer E which is a gastric soluble polymer), in 2nd fluid for dissolution test was almost equivalent to that of the medicament-containing particle (Comparative Example 2-1) free of a functional polymer, and the effect of the functional polymer was not exhibited. In contrast, the medicament-containing particle (Example 6-2) of the present invention showed suppressive effect on the dissolution, and exhibited the effect of the functional polymer.

From Fig. 6-3, the dissolution of the medicament-containing particle (Comparative Example 2-4), which was dry-granulated using a functional polymer (aminoalkylmethacrylate copolymer RS which is a sustained-release polymer), in 2nd fluid for dissolution test was almost equivalent to that of the medicament-containing particle (Comparative Example 2-1) free of a functional polymer, and the effect of the functional polymer was not exhibited. In contrast, the medicament-containing particle (Example 6-3) of the present invention showed a sustained-release dissolution, and exhibited the effect of the functional polymer.

From Fig. 6-4, the dissolution of the medicament-containing particle (Comparative Example 2-5), which was dry-granulated using a functional polymer (hydroxypropylcellulose which is a water-soluble polymer), in 2nd fluid for dissolution test was almost equivalent to that of the medicament-containing particle (Comparative Example 2-1) free of a functional polymer, and the effect of the functional polymer was not exhibited. In contrast, the medicament-containing particle (Example 6-4) of the present invention showed very rapid dissolution, and exhibited the effect of the functional polymer.

From these Examples, it could be confirmed that the medicament-containing particles of the present invention were imparted with the functionality of polymer.

The results obtained using the dissolution test 1st fluid are shown in Fig. 6-5.

From Fig. 6-5, the dissolution of the medicament-containing particle (Comparative Example 2-3), which was dry-granulated using a functional polymer (aminoalkylmethacrylate copolymer E which is a gastric soluble polymer), in dissolution test 1st fluid was almost equivalent to that of the medicament-containing particle (Comparative Example 2-1) free of a functional polymer, and the effect of the functional polymer was not exhibited. In contrast, the medicament-containing particle (Example 6-2) of the present invention showed very rapid dissolution, and exhibited the effect of the functional polymer.

From these Examples, it could be confirmed that the medicament-containing particles of the present invention were imparted with the functionality of polymer.

Example 7

<Effect of polymer (2)>

According to the formulation ratios and charge amounts in Table 21, medicament-containing particles of Examples 7-1 to 7-4 were produced. A jet mill pulverized product of indomethacin (1-(4-chlorobenzoyl)-5-methoxy-2-methyl-1-H-indole-3-acetic acid, hereinafter Compound G) as a medicament and, as polymers, dried methacrylic acid copolymer LD (100 mesh on product) which is an enteric polymer, aminoalkylmethacrylate copolymer E (pulverized by Fitz Mill (screen size: 42 mesh) and 60-100 mesh fraction was used) which is a gastric soluble polymer, aminoalkylmethacrylate copolymer RS (100 mesh on product) which is a sustained-release polymer, and hydroxypropylcellulose (HPC-L) (100-150 mesh fraction) which is a water-soluble polymer were weighed, and they were charged in a high shear granulator (vertical granulator, VG) (FM-VG-05, volume: 5 L, manufactured by POWREX CORPORATION) each in a powdery condition. Under the preparation conditions shown in Table 22, they were granulated for 29 - 61 min while spraying purified water or 95% ethanol aqueous solution (solvent), and fluidized-bed dried by using multiplex MP-01 (manufactured by POWREX CORPORATION) to give Compound G-containing particles. The obtained particles were confirmed to be hollow, and the diameter of the hollow is shown in Table 37-2.

The particle size distribution of the polymers used was measured by a laser diffraction particle size analyzer (manufactured by POWREX CORPORATION, Particle Viewer), and the particle size distribution of the medicament used was measured by a laser diffraction particle size analyzer (manufactured by Shimadzu Corporation, SALD-3000J). The values are shown in Table 23-1.

[Table 21]

| | Example 7-1 | | Example 7-2 | | Example 7-3 | | Example 7-4 | |
|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) |
| Compound G | 80 | 560 | 80 | 560 | 80 | 560 | 90 | 630 |
| dried methacrylic acid copolymer LD (100 mesh on) | 20 | 140 | | | | | | |
| aminoalkylmethacrylate copolymer E (60-100 mesh) | | | 20 | 140 | | | | |
| aminoalkylmethacrylate copolymer RS (100 mesh on) | | | | | 20 | 140 | | |
| hydroxypropylcellulose (100-150 mesh) | | | | | | | 10 | 70 |
| (95% ethanol) | (44) | (305) | (41) | (285) | (55) | (385) | | |
| (purified water) | | | | | | | (24) | (170) |
| Total | 100 | 700 | 100 | 700 | 100 | 700 | 100 | 700 |
| In the Table, the formulation ratio (%) is in wt%. | | | | | | | | |

[Table 22]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 29-61 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 23-1]

| | particle size distribution ($\mu$m) | | |
|---|---|---|---|
| | D10 | D50 | D90 |
| Compound G | 0.8 | 3.1 | 8.1 |
| dried methacrylic acid copolymer LD (100 mesh on) | 54.0 | 131.2 | 212.3 |
| aminoalkylmethacrylate copolymer E (60-100 mesh) | 94.2 | 158.9 | 228.2 |
| aminoalkylmethacrylate copolymer RS (100 mesh on) | 127.2 | 194.6 | 281.3 |
| hydroxypropylcellulose (HPC-L) (100-150 mesh) | 88.2 | 138.4 | 202.6 |

The particle size distribution of the produced particles was measured by Particle Viewer (manufactured by POWREX CORPORATION). The results are shown in Table 23-2.

[Table 23-2]

| Example | particle size distribution ($\mu$m) | | | particle size distribution width D90/D10 |
|---|---|---|---|---|
| | D10 | D50 | D90 | |
| 7-1 | 165 | 256 | 356 | 2.2 |
| 7-2 | 150 | 224 | 318 | 2.1 |
| 7-3 | 232 | 322 | 446 | 1.9 |
| 7-4 | 141 | 214 | 308 | 2.2 |

(Experimental Example 4)

Using the medicament-containing particles produced in Examples 7-1 to 7-4, a dissolution test was performed by the following steps. As a test solution, 2nd fluid for dissolution test (about pH 7) of the Japanese Pharmacopoeia, 15th Edition, was used.

<Preparation of standard solution>

Reference standard of Compound G (about 30 mg) was precisely weighed, and water/acetonitrile (1/1) (about 70 mL) was added. The mixture was dissolved by ultrasonic irradiation for 5 min, and water/acetonitrile (1/1) was added to accurately 100 mL. This solution (2 mL) was accurately weighed and water/acetonitrile (1/1) was added to accurately 20 mL to give a standard solution.

<Preparation of sample solution>

Medicament-containing particles in an amount corresponding to 25 mg of Compound G was precisely weighed and used as a sample. Using a test solution (900 mL) and according to Dissolution Test Method 2 of the Japanese Pharmacopoeia, 15th Edition, the test was performed at 50 rpm. At 5, 10, 15, 30, 45, 60, 120, 180, 240 and 360 min from the start of the dissolution test, the eluate (5 mL) was sampled. The eluate was filtered through a membrane filter (DISMIC-13HP manufactured by ADVANTEC Co., Ltd., 13 mm) having a pore size of 0.20 $\mu$m or less. The initial filtrate (about 3 mL) was removed, the next filtrate was measured by HPLC, and the dissolution rate was calculated.

<HPLC measurement conditions>

detector: ultraviolet absorption spectrophotometer
measurement wavelength: 320 nm
column: Waters ACQUITY UPLC C18 2.1 mm$\times$30 mm 1.7 $\mu$m
column temperature: 40°C
flow rate: 0.5 mL/mim (A: 0.25 mL/mim, B: 0.25 mL/mim)
injection volume: 5 $\mu$L
sample cooler: 25°C
syringe washing: water/acetonitrile mixed solution = 1/1
mobile phase: A: diluted phosphoric acid (1→1000)
B: acetonitrile
The results obtained using the 2nd fluid for dissolution test are shown in Fig. 7.
From Fig. 7, medicament-containing particles (Examples 7-1 and 7-4) using functional polymers soluble in the 2nd fluid for dissolution test (dried methacrylic acid copolymer LD which is an enteric polymer, and hydroxypropylcellulose which is a water-soluble polymer) showed very rapid dissolution, and the medicament-containing particles (Examples 7-2 and 7-3) using functional polymers hardly soluble in the 2nd fluid for dissolution test (aminoalkylmethacrylate copolymer RS which is a sustained-release polymer, and aminoalkylmethacrylate copolymer E which is a gastric soluble polymer) showed a sustained release dissolution profile.
From these Examples, it could be confirmed that the medicament-containing particles of the present invention were imparted with the functionality of polymer.

Example 8

<Low content medicament particles>

As for Example 8-1, according to the formulation ratios and charge amounts in Table 24, a jet mill pulverized product of Compound A as a medicament, hydroxypropylcellulose (HPC-L) (100-150 mesh fraction), which is a water-soluble polymer, as a polymer, and other additive shown in Table 24 in powder were charged in a high shear granulator (vertical granulator, VG) FM-VG-05 (volume: 5 L, manufactured by POWREX CORPORATION). Under the preparation conditions shown in Table 25, they were granulated for 55 min while spraying 95% ethanol aqueous solution (solvent), and fluidized-bed dried by using multiplex MP-01 (manufactured by POWREX CORPORATION) to give Compound A-containing particle. The obtained particle was confirmed to be hollow, and the diameter of the hollow is shown in Table 37-2.
The particle size distribution of the polymer used was measured by a laser diffraction particle size analyzer (manufactured by POWREX CORPORATION, Particle Viewer), and the particle size distribution of the medicament and other additive used was measured by a laser diffraction particle size analyzer (manufactured by Shimadzu Corporation, SALD-3000J). The values are shown in Table 26-1.

[Table 24]

|  | Example 8-1 | |
| --- | --- | --- |
|  | formulation ratio (%) | charge amount (g) |
| Compound A | 1.0 | 7 |
| D-mannitol (JM pulverized product) | 78.8 | 553 |
| hydroxypropylcellulose (100-150 mesh) | 20.0 | 140 |
| aerosil | 0.2 | 1.4 |

(continued)

| | Example 8-1 | |
|---|---|---|
| | formulation ratio (%) | charge amount (g) |
| (95% ethanol) | (40) | (280) |
| Total | 100 | 701.4 |
| In the Table, the formulation ratio (%) is in wt%. | | |

[Table 25]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 55 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 26-1]

| | particle size distribution (μm) | | |
|---|---|---|---|
| | D10 | D50 | D90 |
| Compound A | 0.7 | 2.7 | 5.6 |
| hydroxypropylcellulose (100-150 mesh) | 88.2 | 138.4 | 202.6 |
| D-mannitol (JM pulverized product) | 0.5 | 2.0 | 4.2 |
| aerosil | N.P. | N.P. | N.P. |
| mixed powder of Compound A, D-mannitol (JM pulverized product) and aerosil | 0.6 | 2.2 | 4.7 |
| N.P.: Not Performed | | | |

The particle size distribution of the produced particle was measured by Particle Viewer (manufactured by POWREX CORPORATION). The results are shown in Table 26-2 and Fig. 8-1.

[Table 26-2]

| Example | particle size distribution (μm) | | | particle size distribution width D90/D10 |
|---|---|---|---|---|
| | D10 | D50 | D90 | |
| 8-1 | 139 | 196 | 259 | 1.9 |

As shown in Fig. 8-1, a medicament-containing particle having a very narrow particle size distribution width could be produced even when the medicament content was very low.

The appearance of the produced particle was observed under a scanning electron microscope (S-3400N manufactured by Hitachi, Ltd.). The appearance of the spherical particle is shown in Fig. 8-2. As observed in Fig. 8-2, a spherical particle having extremely high sphericity could be produced.

Comparative Example 3-1

Particles were produced by the method disclosed in patent document 3. Citric acid mosapride (250 g), D-mannitol (PEARLITOL 50C, 750 g) and polyvinylpyrrolidone (plasdone K29-32, 250 g) were charged in a high shear granulator (vertical granulator, VG) FM-VG-05 (volume: 5 L, manufactured by POWREX CORPORATION), and thoroughly mixed. While agitating the mixture with blade (rotation speed 400 rpm) and chopper (rotation speed 3000 rpm), purified water (130 g) was sprayed at a rate of 8 g/min, and the mixture was granulated for 20 min. After granulation, it was fluidized-bed dried by multiplex MP-01 (manufactured by POWREX CORPORATION) to give medicament-containing particles. The appearance and cross-section of the produced particles were observed under a scanning electron microscope (S-3400N manufactured by Hitachi, Ltd.). The appearance of the particles is shown in Fig. 9-1, and the cross-section is shown in Fig. 9-2. The obtained particles were neither spherical nor hollow. The strength of the particles was 1.7 (MPa).

From the above, it was found that the process for preparation disclosed in patent document 3 cannot produce a medicament-containing hollow particle.

Comparative Example 3-2

Particles were produced by the method disclosed in patent document 4. A micronized product of Compound A (133 g), low-substituted hydroxypropylcellulose (LH-31, 347 g), lactose hydrate (Pharmatose 200M, 87 g) and hydroxypropylcellulose (HPC-L, 33 g) were charged in a high shear granulator (vertical granulator, VG) FM-VG-05 (volume: 5 L, manufactured by POWREX CORPORATION), and thoroughly mixed. While agitating the mixture with blade (rotation speed 400 rpm) and chopper (rotation speed 3000 rpm), 95% ethanol solution (380 g) was added dropwise, and the mixture was granulated for 28 min. After granulation, it was fluidized-bed dried by multiplex MP-01 (manufactured by POWREX CORPORATION) to give medicament-containing particles. The produced particles were observed for the appearance under a scanning electron microscope (S-3400N manufactured by Hitachi, Ltd.) and found to partly contain spherical particles (Fig. 9-3). The inner structure of the spherical particles was non-destructively observed by a benchtop micro-CT (manufactured by SKYSCAN, SKYSCAN1172), and found to be not hollow (Fig. 9-4). The particle strength was 2.2 (MPa).

From the above, it was found that the process for preparation disclosed in patent document 4 cannot produce a medicament-containing hollow particle.

Comparative Example 3-3

Particles were produced by the method disclosed in non-patent document 1. An acetaminophen sample mill pulverized product (350 g), D-mannitol (PEARLITOL 50C, 301 g) and low-substituted hydroxypropylcellulose (LH-21, 35 g) were charged in a high shear granulator (vertical granulator, VG) FM-VG-05 (volume: 5 L, manufactured by POWREX CORPORATION), and thoroughly mixed. While agitating the mixture with blades (400 rpm) and choppers (3000 rpm), a granulation liquid obtained by dissolving hydroxypropylcellulose (HPC-L, 7 g) in purified water (126 g) was added dropwise, and the mixture was granulated for 5 min. After granulation, it was fluidized-bed dried by multiplex MP-01 (manufactured by POWREX CORPORATION) to give medicament-containing particles. The appearance of the produced particle was observed under a scanning electron microscope (S-3400N manufactured by Hitachi, Ltd.) to find no spherical particles (Fig. 9-5). The inner structure of the spherical particle was non-destructively observed by a benchtop micro-CT (manufactured by SKYSCAN, SKYSCAN1172), and found to be not hollow (Fig. 9-6). The particle strength was 1.6 (MPa).

From the above, it was found that the process for preparation disclosed in non-patent document 1 cannot produce a medicament-containing hollow particle.

The particle size distribution of the medicament used was measured by a laser diffraction particle size analyzer (manufactured by Shimadzu Corporation, SALD-3000J), and the particle size distribution of the polymer and other additive used were measured by a laser diffraction particle size analyzer (manufactured by POWREX CORPORATION, Particle Viewer). The values are shown in Table 27.

[Table 27]

| Comparative Example 3-1 | particle size distribution (μm) | | |
| --- | --- | --- | --- |
| | D10 | D50 | D90 |
| mosapride citrate | 2.3 | 9.2 | 30.6 |
| polyvinylpyrrolidone | 23.2 | 70.5 | 137.8 |

(continued)

| Comparative Example 3-1 | particle size distribution (μm) | | |
| --- | --- | --- | --- |
| | D10 | D50 | D90 |
| D-mannitol (PEARLITOL 50C) | 6.9 | 47.0 | 125.2 |
| mixed powder of mosapride citrate and D-mannitol (PEARLITOL 50C) | 11.3 | 45.2 | 96.6 |

| Comparative Example 3-2 | particle size distribution (μm) | | |
| --- | --- | --- | --- |
| | D10 | D50 | D90 |
| Compound A | 3.2 | 13.3 | 40.8 |
| hydroxy propyl cellulose | 46.5 | 128.6 | 214.1 |
| low-substituted hydroxypropylcellulose (LH-31) | 8.8 | 20.4 | 38.6 |
| lactose (Pharmatose 200M) | 4.0 | 48.7 | 129.0 |
| mixed powder of Compound A, low-substituted hydroxypropylcellulose (LH-31) and lactose (Pharmatose 200M) | 8.6 | 22.1 | 51.7 |

| Comparative Example 3-3 | particle size distribution (μm) | | |
| --- | --- | --- | --- |
| | D10 | D50 | D90 |
| acetaminophen | 2.8 | 11.6 | 46.1 |
| hydroxypropylcellulose | 46.5 | 128.6 | 214.1 |
| D-mannitol (PEARLITOL 50C) | 6.9 | 47.0 | 125.2 |
| low-substituted hydroxypropylcellulose (LH-21) | 6.8 | 51.5 | 121.2 |
| mixed powder of acetaminophen, D-mannitol (PEARLITOL 50C) and low-substituted hydroxypropylcellulose (LH-21) | 8.0 | 23.7 | 66.0 |

Example 9

<Particle size of medicament>

According to the formulation ratios and charge amounts in Table 28, a jet mill pulverized product of Compound A having different average particle size as a medicament and a particle size controlled product of hydroxypropylcellulose (HPC-L) (100-165 mesh fraction) as a functional polymer in powder were added at 10% relative to the charge amount. Using a high shear granulator (vertical granulator, VG) (FM-VG-05, volume: 5 L) and under the preparation conditions shown in Table 29, the mixture was granulated for 30 min while spraying 50% ethanol aqueous solution or purified water, and fluidized-bed dried by multiplex FD-MP-01 to give Compound A-containing particles of Examples 9-1 and 9-2. The obtained particles were confirmed to be hollow, and the diameter of the hollow is shown in Table 37-2.

The particle size distribution of the polymer used was measured by a laser diffraction particle size analyzer (manufactured by POWREX CORPORATION, Particle Viewer), and the particle size distribution of the medicament used was measured by a laser diffraction particle size analyzer (manufactured by Shimadzu Corporation, SALD-3000J). The values are shown in 30-1. The formulation of Example 1-1 is shown in Table 28, and the particle size distribution of the medicament, polymer and medicament-containing particles is described in Tables 30-1 and 30-2.

[Table 28]

| | Example 1-1 | | Example 9-1 | | Example 9-2 | |
| --- | --- | --- | --- | --- | --- | --- |
| | formulation ratio (%) | charge amount (g) | formulation ratio (%) | charge amount (g) | formulation ratio (%) | charge amount (g) |
| Compound A (D50: 2.7 μm) | 90 | 630 | - | - | - | - |

(continued)

| | Example 1-1 | | Example 9-1 | | Example 9-2 | |
|---|---|---|---|---|---|---|
| | formulation ratio (%) | charge amount (g) | formulation ratio (%) | charge amount (g) | formulation ratio (%) | charge amount (g) |
| Compound A (D50: 6.9 μm) | - | - | 90 | 630 | - | - |
| Compound A (D50: 9.9 μm) | - | - | - | - | 90 | 630 |
| hydroxypropylcellulose (100-165 mesh) | 10 | 70 | 10 | 70 | 10 | 70 |
| (50% ethanol) | (23) | (160) | - | - | - | - |
| (purified water) | - | - | (18) | (125) | (13) | (90) |
| Total | 100 | 700 | 100 | 700 | 100 | 700 |
| In the Table, the formulation ratio (%) is in wt%. | | | | | | |

[Table 29]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 30 | | spray (10 g/min) | 400 | 3,000 |
| dryer | multiplex MP-01 inlet air temperature; 70°C outlet air temperature; completed at 35°C | | | | |

[Table 30-1]

| | particle size distribution (μm) | | |
|---|---|---|---|
| | D10 | D50 | D90 |
| Compound A (D50: 2.7 μm) | 0.7 | 2.7 | 5.6 |
| Compound A (D50: 6.9 μm) | 1.0 | 6.9 | 26.8 |
| Compound A (D50: 9.9 μm) | 1.2 | 9.9 | 42.9 |
| hydroxypropylcellulose (100-165 mesh) | 81.2 | 137.8 | 264.3 |

The particle size distribution of the produced particles was measured by Particle Viewer (manufactured by POWREX CORPORATION). The results are shown in Table 30-2.

[Table 30-2]

| Example | particle size distribution (μm) | | | particle size distribution width D90/D10 |
|---|---|---|---|---|
| | D10 | D50 | D90 | |
| 1-1 | 115 | 175 | 239 | 2.1 |
| 9-1 | 78 | 164 | 251 | 3.2 |
| 9-2 | 54 | 146 | 224 | 4.1 |

The appearance and cross-section of the produced particles were observed under a scanning electron microscope (S-3400N manufactured by Hitachi, Ltd.). As for Examples 9-1 and 9-2, the appearance of the spherical particles is shown in Figs. 10-1 and 10-2. As observed in Figs. 10-1 and 10-2, medicament-containing particles could be produced.

Example 10

<Particle size of polymer>

According to the formulation ratios and charge amounts in Table 31, medicament-containing particles of Examples 10-1 to 10-4 were produced. A jet mill pulverized product of Compound A as a medicament and various fractions of hydroxypropylcellulose, which is a water-soluble polymer, as a polymer in powder were charged in a high shear granulator (vertical granulator, VG) FM-VG-05 (volume: 5 L, manufactured by POWREX CORPORATION). Under the preparation conditions shown in Table 32, they were granulated for 29-39 min while spraying 50% ethanol aqueous solution (solvent), and fluidized-bed dried by multiplex MP-01 (manufactured by POWREX CORPORATION) to give Compound A-containing particles. The obtained particles were confirmed to be hollow, and the diameter of the hollow is shown in Table 37-2.
The particle size distribution of the polymer used was measured by a laser diffraction particle size analyzer (manufactured by POWREX CORPORATION, Particle Viewer), and the particle size distribution of the medicament used was measured by a laser diffraction particle size analyzer (manufactured by Shimadzu Corporation, SALD-3000J). The values are shown in Table 33-1. The formulation of Example 1-1 is shown in Table 31, and the particle size distribution of the medicament, polymer and medicament-containing particles is described in Tables 33-1 and 33-2.

[Table 31]

| | Example 10-1 | | Example 1-1 | | Example 10-2 | | Example 10-3 | | Example 10-4 | |
|---|---|---|---|---|---|---|---|---|---|---|
| | formulation ratio (%) | charge amount (g) | formulation ratio (%) | charge amount (g) | formulation ratio (%) | charge amount (g) | formulation ratio (%) | charge amount (g) | formulation ratio (%) | charge amount (g) |
| Compound A | 90 | 630 | 90 | 630 | 90 | 630 | 90 | 630 | 60 | 630 |
| hydroxypropylcellulose (100 mesh on) | 10 | 70 | - | - | - | - | - | - | - | - |
| hydroxypropylcellulose (100-165 mesh) | - | - | 10 | 70 | - | - | - | - | - | - |
| hydroxypropylcellulose (165-200 mesh) | - | - | - | - | 10 | 70 | - | - | - | - |
| hydroxypropylcellulose (200-325 mesh) | - | - | - | - | - | - | 10 | 70 | - | - |
| hydroxypropylcellulose (325 mesh pass) | - | - | - | - | - | - | - | - | 10 | 70 |
| (50% ethanol) | (21) | (150) | (23) | (160) | (21) | (150) | (21) | (145) | (21) | (145) |
| Total | 100 | 700 | 100 | 700 | 100 | 700 | 100 | 700 | 100 | 700 |
| In the Table, the formulation ratio (%) is in wt%. | | | | | | | | | | |

[Table 32]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 29-39 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 33-1]

| | particle size distribution (μm) | | |
|---|---|---|---|
| | D10 | D50 | D90 |
| Compound A | 0.7 | 2.7 | 5.6 |
| hydroxypropylcellulose (100 mesh on) | 130.8 | 197.1 | 281.5 |
| hydroxypropylcellulose (100-165 mesh) | 81.2 | 137.8 | 264.3 |
| hydroxypropylcellulose (165-200 mesh) | 61.7 | 98.0 | 175.8 |
| hydroxypropylcellulose (200-325 mesh) | 39.3 | 69.8 | 108.9 |
| hydroxypropylcellulose (325 mesh pass) | 15.4 | 34.6 | 61.0 |

The particle size distribution of the produced particles was measured by Particle Viewer (manufactured by POWREX CORPORATION). The results are shown in Table 33-2.

[Table 33-2]

| Example | particle size distribution (μm) | | | particle size distribution width D90/D10 |
|---|---|---|---|---|
| | D10 | D50 | D90 | |
| 10-1 | 164 | 295 | 454 | 2.8 |
| 1-1 | 115 | 175 | 239 | 2.1 |
| 10-2 | 93 | 151 | 222 | 2.4 |
| 10-3 | 72 | 113 | 162 | 2.2 |
| 10-4 | 31 | 63 | 127 | 4.1 |

Example 11

<Study of water-soluble polymer in different grades>

As for Example 11-1, according to the formulation ratios and charge amounts in Table 34, a jet mill pulverized product of Compound A as a medicament and hydroxypropylcellulose HPC-SSL (100-140 mesh fraction) as a polymer in powder were charged in a high shear granulator (vertical granulator, VG) (FM-VG-05, volume: 5 L, manufactured by POWREX CORPORATION). Under the preparation conditions shown in Table 35, the mixture was granulated for 19 min while spraying 80% ethanol aqueous solution (solvent), and fluidized-bed dried by multiplex MP-01 (manufactured by POWREX CORPORATION) to give Compound A-containing hollow particles having high sphericity. The obtained particles were confirmed to be hollow, and the particle size of the hollow is shown in Table 37-2.

The particle size distribution of the polymer used was measured by a laser diffraction particle size analyzer (manufactured by POWREX CORPORATION, Particle Viewer), and the particle size distribution of the medicament used was measured by a laser diffraction particle size analyzer (manufactured by Shimadzu Corporation, SALD-3000J). The values are shown in Table 36-1.

[Table 34]

|  | Example 11-1 | |
|---|---|---|
|  | formulation ratio (%) | charge amount (g) |
| Compound A | 70 | 420 |
| hydroxypropylcellulose (HPC-SSL 100-140 mesh) | 30 | 180 |
| (80% ethanol) | (18) | (110) |
| Total | 100 | 600 |
| In the Table, the formulation ratio (%) is in wt%. | | |

[Table 35]

| process | time (min) | polymer addition method | solvent addition method | blade rotation speed (rpm) | chopper rotation speed (rpm) |
|---|---|---|---|---|---|
| mixing, granulator | vertical granulator FM-VG-05 | | | | |
| pre-mixing | 3 | powder addition | - | 400 | 3,000 |
| granulation | 19 | | spray (8 g/min) | 400 | 3,000 |
| dryer | multiplex MP-01 inlet air temperature; 70°C outlet air temperature; completed at 35°C | | | | |
| In the Table, (min) shows (minutes). | | | | | |

[Table 36-1]

|  | particle size distribution (μm) | | |
|---|---|---|---|
|  | D10 | D50 | D90 |
| Compound A | 0.7 | 2.7 | 5.6 |
| hydroxypropylcellulose (HPC-SSL 100-140 mesh) | 94.8 | 155.9 | 225.9 |

The particle size distribution of the produced particles was measured by Particle Viewer (manufactured by POWREX CORPORATION). The results are shown in Table 36-2.

[Table 36-2]

| Example | particle size distribution (μm) | | | particle size distribution width D90/D10 |
|---|---|---|---|---|
|  | D10 | D50 | D90 |  |
| 11-1 | 130 | 192 | 263 | 2.0 |

Experimental Example 5

The medicament-containing particles (hollow particles) obtained in Examples 1 to 4 and 6 to 11 were evaluated for the aspect ratio, particle shell strength, particle size of medicament-containing particle, diameter of hollow, shell thickness, percentage of the shell thickness, volume ratio of the hollow relative to the volume of whole particle, and surface smoothness, according to the above-mentioned test methods and/or calculation methods. The results are shown in Table 37-1 and Table 37-2.

The particle size distribution ratio (D50/D50) of the polymers and medicaments, the particle size distribution ratio (D10/D90) of the polymers and medicaments, the particle size distribution ratio (D50/D50) of the polymer and a mixed powder of the medicament and other additive, and the particle size distribution ratio (D10/D90) of the polymer and a mixed powder of the medicament and other additive, which were used in the Examples and Comparative Examples, are shown in Tables 38-1 to 38-3.

[Table 37-1]

| Example | volume ratio of hollow (%) | particle size of medicament containing particle (μm) | shell thickness (μm) | diameter of hollow (μm) | percentage of shell thickness (%) | aspect ratio | surface smoothness (visually observed) | particle shell strength (MPa) |
|---|---|---|---|---|---|---|---|---|
| 1-1 | 2.7 | 202 | 71 | 59 | 70 | 1.12 | +++ | 8.1 |
| 1-2 | 3.2 | 158 | 54 | 49 | 69 | 1.06 | +++ | 5.2 |
| 1-3 | N.P. | N.P. | N.P. | N.P. | N.P. | N.P. | +++ | N.P. |
| 1-4 | N.P. | N.P. | N.P. | N.P. | N.P. | N.P. | +++ | N.P. |
| 1-5 | 3.3 | 207 | 71 | 66 | 68 | 1.17 | +++ | 4.0 |
| 1-6 | N.P. | N.P. | N.P. | N.P. | N.P. | N.P. | +++ | N.P. |
| 1-7 | 12.3 | 247 | 62 | 123 | 51 | 1.16 | +++ | 6.1 |
| 2-1 | 3.7 | 201 | 67 | 67 | 67 | 1.14 | +++ | 5.2 |
| 1-1 | 2.7 | 202 | 71 | 59 | 70 | 1.12 | +++ | 8.1 |
| 2-2 | 4.6 | 195 | 63 | 69 | 65 | 1.20 | +++ | 4.5 |
| 2-3 | 7.3 | 130 | 38 | 54 | 59 | 1.30 | +++ | 9.0 |
| 1-1 | 2.7 | 202 | 71 | 59 | 70 | 1.12 | +++ | 8.1 |
| 3-1 | 22.3 | 175 | 35 | 106 | 40 | 1.28 | +++ | 7.8 |
| 3-2 | 5.7 | 135 | 42 | 51 | 62 | 1.21 | +++ | 5.7 |
| 3-3 | 19.6 | 148 | 31 | 85 | 42 | 1.42 | ++ | 7.1 |
| 3-4 | 7.3 | 142 | 41 | 59 | 59 | 1.32 | +++ | 7.6 |
| 3-5 | 18.6 | 190 | 41 | 108 | 43 | 1.18 | +++ | 7.5 |
| 3-6 | 11.9 | 104 | 27 | 51 | 51 | 1.27 | +++ | 4.9 |
| 3-7 | 27.7 | 120 | 21 | 78 | 35 | 1.14 | +++ | 6.6 |
| 4-1 | 2.9 | 290 | 101 | 88 | 70 | 1.06 | +++ | 3.2 |
| 4-2 | N.P. | N.P. | N.P. | N.P. | N.P. | N.P. | +++ | N.P. |
| N.P.: Not Performed | | | | | | | | |

[Table 37-2]

| Example | volume ratio of hollow (%) | particle size of medicament containing particle (μm) | shell thickness (μm) | diameter of hollow (μm) | percentage of shell thickness (%) | aspect ratio | surface smoothness (visually observed) | particle shell strength (MPa) |
|---|---|---|---|---|---|---|---|---|
| 3-7 | 27.7 | 120 | 21 | 78 | 35 | 1.14 | +++ | 6.6 |
| 6-1 | 22.3 | 177 | 35 | 107 | 39 | 1.09 | +++ | 8.3 |
| 6-2 | 21.6 | 283 | 57 | 170 | 40 | 1.18 | +++ | 6.6 |
| 6-3 | 15.7 | 350 | 81 | 189 | 46 | 1.16 | +++ | 7.3 |
| 6-4 | 12.5 | 213 | 54 | 106 | 50 | 1.15 | +++ | 5.6 |
| 7-1 | 16.5 | 268 | 60 | 147 | 45 | 1.22 | +++ | 8.1 |
| 7-2 | 19.5 | 196 | 42 | 113 | 42 | 1.06 | +++ | 6.2 |
| 7-3 | 14.6 | 296 | 70 | 156 | 47 | 1.08 | +++ | 7.9 |
| 7-4 | 9.9 | 273 | 74 | 126 | 54 | 1.15 | +++ | 4.7 |
| 8-1 | 14.4 | 177 | 42 | 93 | 48 | 1.13 | +++ | 4.3 |
| 1-1 | 2.7 | 202 | 71 | 59 | 70 | 1.12 | +++ | 8.1 |
| 9-1 | 4.4 | 200 | 65 | 70 | 65 | 1.19 | ++ | 4.9 |
| 9-2 | 4.9 | 196 | 62 | 71 | 64 | 1.25 | + | 5.2 |
| 10-1 | 6.5 | 358 | 107 | 145 | 60 | 1.23 | +++ | 5.8 |
| 1-1 | 2.7 | 202 | 71 | 59 | 70 | 1.12 | +++ | 8.1 |
| 10-2 | N.P. | N.P. | N.P. | N.P. | N.P. | N.P. | +++ | N.P. |
| 10-3 | 2.0 | 108 | 39 | 29 | 73 | 1.15 | +++ | 4.5 |
| 10-4 | 1.6 | 64 | 24 | 16 | 76 | 1.18 | + | 2.1 |
| 11-1 | 8.1 | 179 | 51 | 77 | 57 | 1.26 | +++ | 5.5 |
| N.P.: Not Performed | | | | | | | | |

[Table 38-1]

| Example | particle size distribution ratio (D50/D50) of polymer and medicament | particle size distribution ratio (D10/D90) of polymer and medicament | particle size distribution ratio (D50/D50) of polymer and mixed powder of medicament and other additive | particle size distribution ratio (D10/D90) of polymer and mixed powder of medicament and other additive |
|---|---|---|---|---|
| 1-1 | 51.0 | 14.5 | | |
| 1-2 | 65.3 | 18.7 | | |
| 1-3 | 43.1 | 15.6 | | |
| 1-4 | 30.0 | 6.4 | | |
| 1-5 | 43.1 | 12.9 | | |
| 1-6 | 55.1 | 16.2 | | |
| 1-7 | 55.1 | 16.2 | | |
| 2-1 | 51.0 | 14.5 | | |
| 1-1 | 51.0 | 14.5 | | |
| 2-2 | 51.0 | 14.5 | | |
| 2-3 | 51.0 | 14.5 | | |
| 1-1 | 51.0 | 14.5 | | |
| 3-1 | 48.6 | 13.1 | | |
| 3-2 | 51.3 | 13.2 | | |
| 3-3 | 70.6 | 20.9 | | |
| 3-4 | 53.4 | 17.7 | | |
| 3-5 | 53.7 | 20.0 | | |
| 3-6 | 63.3 | 20.1 | | |
| 3-7 | 26.0 | 5.9 | | |
| 4-1 | 66.5 | 12.5 | 59.6 | 13.1 |
| 4-2 | 46.5 | 11.9 | 31.7 | 7.1 |

[Table 38-2]

| Example | particle size distribution ratio (D50/D50) of polymer and medicament | particle size distribution ratio (D10/D90) of polymer and medicament | particle size distribution ratio (D50/D50) of polymer and mixed powder of medicament and other additive | particle size distribution ratio (D10/D90) of polymer and mixed powder of medicament and other additive |
|---|---|---|---|---|
| 3-7 | 26.0 | 5.9 | | |
| 6-1 | 37.5 | 9.8 | | |
| 6-2 | 58.9 | 16.8 | | |
| 6-3 | 72.1 | 22.7 | | |
| 6-4 | 51.3 | 15.8 | | |
| 7-1 | 41.8 | 6.6 | | |
| 7-2 | 50.6 | 11.6 | | |
| 7-3 | 62.0 | 15.6 | | |
| 7-4 | 44.1 | 10.8 | | |
| 8-1 | 51.3 | 15.8 | 63.2 | 18.7 |
| 1-1 | 51.0 | 14.5 | | |
| 9-1 | 20.0 | 3.0 | | |
| 9-2 | 13.9 | 1.9 | | |
| 10-1 | 73.0 | 23.4 | | |
| 1-1 | 51.0 | 14.5 | | |
| 10-2 | 36.3 | 11.0 | | |
| 10-3 | 25.9 | 7.0 | | |
| 10-4 | 12.8 | 2.8 | | |
| 11-1 | 57.7 | 16.9 | | |

[Table 38-3]

| Comparative Example | particle size distribution ratio (D50/D50) of polymer and medicament | particle size distribution ratio (D10/D90) of polymer and medicament | particle size distribution ratio (D50/D50) of polymer and mixed powder of medicament and other additive | particle size distribution ratio (D10/D90) of polymer and mixed powder of medicament and other additive |
|---|---|---|---|---|
| 2-1 2-2 | 17.4 | 4.0 | | |
| 2-3 | 4.7 | 1.3 | | |
| 2-4 | 40.0 | 6.3 | | |
| 2-5 | 47.6 | 8.3 | | |
| 3-1 | 7.7 | 0.8 | 1.6 | 0.2 |
| 3-2 | 9.7 | 1.1 | 5.8 | 0.9 |
| 3-3 | 11.1 | 1.0 | 5.4 | 0.7 |

Industrial Applicability

According to the present invention, a spherical particle having sufficient strength of the level facilitating processing such as compression, coating and the like, and having a hollow structure with a pharmaceutically useful and desired function can be provided. According to the present invention, moreover, a particle having good fluidity, capable of increasing a medicament content, superior in particle homogeneity, and showing good mixing uniformity with other components can be provided. According to the present invention, furthermore, particles, from those having a small specific gravity to those having a large specific gravity, can be industrially produced by a single method, and therefore, preparation of a floating particle useful as an intragastric floating preparation and the like, which has a density modified by freely controlling the hollow size, can be expected.

This application is based on an international application PCT/JP2012/071016 based on the Patent Cooperation Treaty, the contents of which are incorporated in full herein.

**Claims**

1. A hollow particle, wherein the particle is composed of a shell and a hollow, the shell comprises a medicament and a polymer, a volume ratio of the hollow relative to the whole particle is 1% - 50%, and a shell thickness of the particle is not less than 15 $\mu$m.

2. The hollow particle according to claim 1, wherein the polymer used as a starting material has an average particle size of not less than 5-fold that of the medicament used as a starting material, or the shell further comprises other additive and the polymer used as a starting material has an average particle size of not less than 5-fold that of a mixed powder of the medicament and the other additive used as a starting material.

3. The hollow particle according to claim 2, which is produced by comprising a step of granulating a powder mixture containing the medicament and the polymer or a powder mixture containing the medicament, the polymer and the other additive, while spraying a solvent capable of dissolving the polymer.

4. The hollow particle according to any one of claims 1 to 4, wherein the hollow has a diameter of not less than 10 $\mu$m.

5. The hollow particle according to any one of claims 2 to 4, which has a medicament content of 0.1 - 96 wt% per 100 wt% and a polymer content of 4 - 50 wt% per 100 wt% of the hollow particle, or a medicament content of 0.1 - 95.9 wt% per 100 wt%, other additive content of 0.1 - 95.9 wt% and a polymer content of 4 - 40 wt% per 100 wt% of the hollow particle.

6. The hollow particle according to any one of claims 1 to 5, wherein the polymer is one or more kinds selected from the group consisting of a water-soluble polymer, a water-insoluble polymer, an enteric polymer, a gastric soluble polymer and a biodegradable polymer.

7. The hollow particle according to claim 6, wherein the water-soluble polymer is selected from the group consisting of methylcellulose, hydroxypropylcellulose, hydroxypropylmethylcellulose, hydroxyethylcellulose, hydroxymethylcellulose, carboxymethylcellulose, polyvinylpyrrolidone, polyvinyl alcohol, copolyvidone, polyethylene glycol, polyvinyl alcohol-acrylic acid-methyl methacrylate copolymer, vinyl acetate-vinylpyrrolidone copolymer, polyvinyl alcohol-polyethylene glycol-graft copolymer, pregelatinized starch, dextrin, dextran, pullulan, alginic acid, gelatin, pectin, and a mixture of one or more kinds thereof, the water-insoluble polymer is selected from the group consisting of ethylcellulose, acetyl cellulose, aminoalkylmethacrylate copolymer RS, ethyl acrylate-methyl methacrylate copolymer dispersion, vinyl acetate resin, and a mixture of one or more kinds thereof, and/or the enteric polymer is selected from the group consisting of hydroxypropylmethylcellulose acetate succinate, hydroxypropylmethylcellulose phthalate, methacrylic acid copolymer L, methacrylic acid copolymer LD, dried methacrylic acid copolymer LD, methacrylic acid copolymer S, methacrylic acid-acrylic acid n-butyl copolymer, and a mixture of one or more kinds thereof.

8. The hollow particle according to any one of claims 2 to 7, wherein said other additive is selected from the group consisting of filler, binder, sweetening agent, corrigent, smell masking agent, flavor, fluidizer, antistatic agent, colorant, disintegrant, lubricant, plasticizer, anticoagulant and coating agent.

9. The hollow particle according to any one of claims 1 to 8, wherein the hollow particle has an aspect ratio of 1.0 - 1.5 and/or a particle shell strength of not less than 2.0 MPa.

10. A pharmaceutical composition comprising a plurality of the hollow particle according to any one of claims 1 to 9.

11. The pharmaceutical composition according to claim 10, wherein the hollow particle has a particle size distribution width (D90/D10) of not more than 6 and/or an average particle size of 50 - 1000 μm.

12. The pharmaceutical composition according to any one of claims 10 or 11, which is in the form of any of granule, tablet and capsule.

13. A process for preparation of the hollow particle according to any one of claims 1, 6 and 7, which comprises a step of granulating a powder mixture containing a medicament and a polymer, while spraying a solvent capable of dissolving the polymer, wherein the polymer in the powder mixture has an average particle size of not less than 5-fold that of medicament.

14. A process for preparation of the hollow particle according to any one of claims 2, 6 and 7, which comprises a step of granulating a powder mixture containing a medicament, a polymer and other additive, while spraying a solvent capable of dissolving the polymer, wherein the polymer in the powder mixture has an average particle size of not less than 5-fold that of the mixed powder of the medicament and other additive.

15. The process for preparation according to claim 13 or 14, wherein the granulation is agitating granulation.

Fig. 1-1

Example 1-1 spherical particles
containing 90% of Compound A (X250)

Fig. 1-2

Example 1-2 spherical particles
containing 90% of Compound B (X250)

## Fig. 1-3

Example 1-3  spherical particles
containing 90% of Compound C (X200)

## Fig. 1-4

Example 1-4  spherical particles
containing 90% of Compound D (X250)

## Fig. 1-5

Example 1-5  spherical particles
containing 90% of Compound E (X200)

Fig. 1-6

Example 1-6 spherical particles
containing 90% of Compound F (X200)

Fig. 1-7

Example 1-1 spherical particle
containing 90% of Compound A (X300)

Fig. 1-8

Example 1-3 spherical particle
containing 90% of Compound C (X200)

Fig. 1-9

Example 1-4 spherical particle
containing 90% of Compound D (X200)

Fig. 1-10

Fig. 2-1

Example 2-1 Compound A-containing
spherical particles (X100)

Fig. 2-2

Example 2-2 Compound A-containing
spherical particles (X200)

Fig. 2-3

Example 2-1  Compound A-containing
spherical particle (X200)

Fig. 2-4

Example 2-2  Compound A-containing
spherical particle (X200)

Fig. 2-5

Example 2-3 Compound A-containing spherical
particle (X200)

Fig. 3

Example 3-5 Compound A-containing spherical
particles X ray CT image

## Fig. 4

Example 5-3  Compound A-containing
particle in tablet

## Fig. 5

Comparative Example 1  medicament-
containing particles X ray CT image

Fig. 6-1

2nd fluid for dissolution test (dried methacrylic acid copolymer LD)

Fig. 6-2

2nd fluid for dissolution test (aminoalkylmethacrylate copolymer E)

Fig. 6-3

2nd fluid for dissolution test
(aminoalkylmethacrylate copolymer RS)

Fig. 6-4

2nd fluid for dissolution test
(hydroxypropylcellulose)

Fig. 6-5

**1st fluid for dissolution test
(aminoalkylmethacrylate copolymer E)**

Fig. 7

**2nd fluid for dissolution test**

Fig. 8-1

Fig. 8-2

Example 8-1 medicament-containing
spherical particle (X500)

Fig. 9-1

Comparative Example 3-1 medicament-
containing particles (X50)

Fig. 9-2

Comparative Example 3-1 medicament-
containing particle (X200)

Fig. 9-3

Comparative Example 3-2 medicament-
containing particles (X100)

Fig. 9-4

Comparative Example 3-2 medicament-
containing particles X ray CT image

Fig. 9-5

Comparative Example 3-3 medicament-
containing particles (X100)

Fig. 9-6

Comparative Example 3-3 medicament-
containing particles X ray CT image

Fig. 10-1

Example 9-1 medicament-containing
particles (X100)

Fig. 10-2

Example 9-2 medicament-containing
particles (X100)

Europäisches
Patentamt

European
Patent Office

Office européen
des brevets

# EUROPEAN SEARCH REPORT

Application Number

EP 20 20 3011

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HAPGOOD K P ET AL: "Agglomeration of hydrophobic powders via solid spreading nucleation", POWDER TECHNOLOGY, ELSEVIER SEQUOIA, LAUSANNE, CH, vol. 188, no. 3, 10 January 2009 (2009-01-10), pages 248-254, XP025716957, ISSN: 0032-5910, DOI: 10.1016/J.POWTEC.2008.05.004 [retrieved on 2008-05-28] * item 2.3 small scale granulation; page 249, left-hand column; table 1 * * page 249, right-hand column; table 2 * * page 251, left-hand column, paragraph 2 * * page 253; figure 6 * | 1-15 | INV. A61K9/50 A61K9/14 A61K47/32 A61K47/34 A61K47/38 A61K9/16 A61K9/20 |
| Y | US 5 057 323 A (NIWA TOSHIYUKI [JP] ET AL) 15 October 1991 (1991-10-15) * column 1, lines 6-9, 28-36, 46-49,64-68 * * column 2, lines 1-7, 48-57 * * column 4; example 1 * | 1-15 | |
| Y | WO 2009/115579 A1 (NAT UNIV IRELAND [IE]; PANDIT ABHAY [IE]; RETHORE GILDAS [IE]; NAIK HE) 24 September 2009 (2009-09-24) * page 1, lines 6-10 * * page 5, lines 22-24 * * page 9, lines 8-10,13-14 * | 1-15 | |

TECHNICAL FIELDS
SEARCHED        (IPC)

A61K

The present search report has been drawn up for all claims

| Place of search | Date of completion of the search | Examiner |
|---|---|---|
| Munich | 10 December 2020 | Raposo, Antonio |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 20 20 3011

This annex lists the patent family members relating to the patent documents cited in  the above-mentioned European search report.
The members are as contained in the European Patent Office EDP file on
The European Patent Office is in no way liable for these particulars which are merely given for the purpose of information.

10-12-2020

| Patent document cited in search report | | Publication date | Patent family member(s) | | Publication date |
|---|---|---|---|---|---|
| US 5057323 | A | 15-10-1991 | JP | H082781 B2 | 17-01-1996 |
| | | | JP | H02250822 A | 08-10-1990 |
| | | | US | 5057323 A | 15-10-1991 |
| | | | US | 5087455 A | 11-02-1992 |
| WO 2009115579 | A1 | 24-09-2009 | EP | 2276475 A1 | 26-01-2011 |
| | | | IE | 20080211 A1 | 25-11-2009 |
| | | | US | 2011123456 A1 | 26-05-2011 |
| | | | US | 2014004251 A1 | 02-01-2014 |
| | | | WO | 2009115579 A1 | 24-09-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000128774 A
- JP 2000072660 A
- WO 2011019043 A
- JP H03130214 A
- JP 2012071016 W

**Non-patent literature cited in the description**

- *Reports of the Mie Prefecture Industrial Research Institute,* 2010, vol. 34, 30-37
- Japanese Pharmacopoeia
- the Japanese Pharmacopoeia